# EUROPÄISCHE PATENTSCHRIFT

(11) **EP 2 809 633 B1**
(45) Veröffentlichungstag und Bekanntmachung des Hinweises auf die Patenterteilung: **30.12.2015**
(21) Anmeldenummer: 13701487.4
(22) Anmeldetag: 30.01.2013
(51) Int. Cl.: C07C 5/48, C07C 7/11, C07C 5/333, C07C 7/08, C07C 11/167

(54) **VERFAHREN ZUR HERSTELLUNG VON BUTADIEN UND/ODER BUTENEN AUS N-BUTAN**
PROCESS FOR PREPARING BUTADIENE AND/OR BUTENES FROM N-BUTANE
PROCÉDÉ DE PRODUCTION DE BUTADIÈNE ET/OU DE BUTÈNES À PARTIR DE N-BUTANE

(30) Priorität: 30.01.2012 EP 12153056
(43) Veröffentlichungstag der Anmeldung: 10.12.2014
(73) Patentinhaber: BASF SE, 67056 Ludwigshafen (DE)
(72) Erfinder: GIESA, Sonja, 64293 Darmstadt (DE); BENFER, Regina, 67122 Altrip (DE)
(86) Internationale Anmeldenummer: PCT/EP2013/051763
(87) Internationale Veröffentlichungsnummer: WO 2013/113743

(56) Entgegenhaltungen:
- WO-A1-2004/007408
- WO-A1-2005/042449
- WO-A1-2005/063657

## Beschreibung

Die Erfindung betrifft Verfahren zur Herstellung von Butadien oder Butenen aus n-Butan.

Butene und Butadien können zum Beispiel durch thermische Spaltung (steam cracking) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Methylallen, C₅-und höheren Kohlenwasserstoffen an.

Unter Butenen werden 1-Buten, Isobuten, 2-Butene und deren Gemische verstanden. Buten-Ströme enthalten beispielsweise 30 bis 80 Gew.-% an Butenen, z. B. 14 Gew.-% 1-Buten, 10 Gew.-% Isobuten, 15,5 Gew.-% trans-2-Buten und 16,5 Gew.-% cis-2-Buten. Erfindungsgemäß werden unter Butenen 1-Buten und 2-Butene verstanden.

Nachteil dieses Verfahrens zur Erzeugung von Butenen und Butadien ist, dass zwangsläufig größere Mengen an unerwünschten Koppelprodukten anfallen. Alternativ können die Butene aus Butan und das Butadien aus n-Buten durch Dehydrierung hergestellt werden.

Aus DE-A 10 2004 059 356 ist es zum Beispiel bekannt, zur Herstellung von Butadien n-Butan als Einsatzstoff einzusetzen. Zur Herstellung des Butadiens wird das n-Butan in einer Dehydrierzone durch nicht-oxidative, katalytische Dehydrierung in einem n-Butan, 1-Buten, 2-Buten, Butadien, Wasserstoff, gegebenenfalls Kohlendioxid und gegebenenfalls Wasserdampf enthaltenden Stoffstrom dehydriert. Das erhaltene Gasgemisch wird abgekühlt und dann direkt in die nächste Dehydrierzone geführt. In einer zweiten Dehydrierzone werden das 1-Buten und 2-Buten weiter zu Butadien dehydriert. Der in der Dehydrierung erhaltene Stoffstrom wird anschließend komprimiert und abgekühlt, um Wasser auszukondensieren. Aus dem Reststrom, der n-Butan, Butadien, Wasserstoff, Kohlendioxid und Wasserdampf enthält, wird durch Extraktivdestillation ein im Wesentlichen Butadien enthaltender Produktstrom abgetrennt.

Ein entsprechendes Verfahren zur Herstellung von Butadien aus n-Butan ist weiterhin auch in DE-A 10 2004 061 514 beschrieben.

Nachteil der hier beschriebenen Verfahren ist, dass die zweite Dehydrierzone unmittelbar auf die erste folgt. Die Zusammensetzung des Eintrittsgases der zweiten Dehydrierstufe wird daher von der Zusammensetzung des Gasstromes aus der ersten Dehydrierstufe vorgegeben und kann nicht optimiert werden. Die neben den C4-Kohlenwasserstoffen enthaltenen Gasbestandteile wie Stickstoff, Dampf, Wasserstoff, Kohlenmonoxid und/oder Kohlendioxid, die zum Teil in der ersten Dehydrierstufe gebildet werden, werden vollständig durch die zweite Dehydrierstufe geleitet. Eine Rückführung des in der ersten Dehydrierstufe nicht umgesetzten Butans erfolgt erst nach Passieren der zweiten Dehydrierstufe und der nachfolgenden Aufarbeitungsstufen. Dabei wird das Butan gemeinsam mit nicht umgesetzten Butenen abgetrennt und in die erste Dehydrierstufe zurückgeführt. Die Rückführung von nicht in der zweiten Dehydrierstufe umgesetzten Butenen in die erste Dehydrierstufe beeinflusst die dortige Butenausbeute nachteilig. Nachteilig ist außerdem, dass das nicht umgesetzte Butan der ersten Dehydrierstufe im Eingangsgasstrom der zweiten Dehydrierstufe ebenfalls nicht abgetrennt bzw. der Butengehalt dieses Stromes durch eine Abreicherung des Butans eingestellt wird.

Eine Abtrennung des in der ersten Dehydrierstufe gebildeten Wasserstoffs und Nutzung zur Bereitstellung der für die Reaktion benötigten Energie durch Umsetzung mit Sauerstoff bei einer Rückführung in diese Stufe ist hier nicht möglich.

In DE-A-10 2004 054 766 ist ein Verfahren zur Herstellung von Butadien aus n-Butan beschrieben, bei dem der Butadien enthaltende Stoffstrom aus der zweistufigen Dehydrierung zunächst abgekühlt wird, um Wasser auszukondensieren. In einer weiteren Kompressionsstufe und Abkühlung wird ein Kondensatstrom, der n-Butan, Butadien und Wasser enthält, erhalten. Aus dem Wasser, n-Butan und Butadien enthaltenden Stoffstrom werden n-Butan und Butadien abgetrennt und anschließend in einen im Wesentlichen aus Butadien bestehenden Produktstrom und einen n-Butan enthaltenden Rückführstrom getrennt.

Nachteil hier ist, dass die C₄-Komponenten von den Inertgasen durch eine mehrstufige Kompression und nachfolgende Kondensation abgetrennt werden. Diese Verfahrensstufe zeichnet sich durch einen hohen Energiebedarf für die Kompression auf bis ca. 30 bar aus. Die C₄-Kondensation erfolgt bei einer Temperatur von 10 °C, somit wird zusätzlich eine Kälteanlage erforderlich. Zudem wird der Produktstrom der ersten Dehydrierung wiederum nach Abkühlen direkt in die zweite Dehydrierung geführt.

WO 2005/063657 A1 betrifft ein Verfahren zur Herstellung von Butadien aus n-Butan durch nicht-oxidative katalytische Dehydrierung und nachfolgende oxidative Dehydrierung in Gegenwart von Sauerstoff in einer zweiten Dehydrierzone, gefolgt von einer destillativen Auftrennung der Produkte und Isomerisierung von 2-Buten zu 1-Buten. Aufgabe der vorliegenden Erfindung ist es, ein Verfahren zur Herstellung eines Butadien enthaltenden Stoffstrorns und optional eines Buten und Butan enthaltenen weiteren Stoffstromes ausgehend von Butan bereitzustellen, das eine optimale Nutzung des eingesetzten Butans und einen optimierten Betrieb einer zweiten Dehydrierstufe erlaubt.

Gelöst wird die Aufgabe durch ein Verfahren zur Herstellung von Butadien aus n-Butan mit den Schritten:
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Butene, Butadien, Wasserstoff, gegebenenfalls Wasserdampf, gegebenenfalls Kohlenstoffoxide und gegebenenfalls Inertgase erhalten wird;
C) Kompression in mindestens einer ersten Kompressionsstufe und Abkühlung des Gasstroms b, wobei mindestens ein Kondensatstrom c1 enthaltend Wasser und ein Stoffstrom c2 enthaltend Butene und Butadien, n-Butan, Wasserstoff, Wasserdampf, gegebenenfalls Kohlenstoffoxide und gegebenenfalls Inertgase erhalten wird;
D) Absorption der Butene und des Butadien, n-Butan, Wasserstoff, Wasserdampf, gegebenenfalls Inertgase und gegebenenfalls Kohlenstoffoxide enthaltenden Stoffstroms c2 mit einem selektiven Lösungsmittel, vorzugsweise einem 80 bis 97 Gew.-% N-Methylpyrrolidon und 3 bis 20 Gew.-% Wasser enthaltenen Gemisch, wobei ein selektives Lösungsmittel, vorzugsweise N-Methylpyrrolidon, Wasser sowie Butene, Butadien, Butan und gegebenenfalls Kohlendioxid enthaltender Stoffstrom d1 und ein Wasserstoff und gegebenenfalls Inertgase und Butan enthaltender Stoffstrom d2 erhalten werden;
E) Extraktivdestillation des selektiven Lösungsmittels, vorzugsweise N-Methylpyrrolidon, Wasser sowie Butene, Butadien, Butan und gegebenenfalls Kohlenstoffoxide enthaltenden Stoffstrom d1 mit einem selektiven Lösungsmittel, vorzugsweise einem 80 bis 97 Gew.-% N-Methylpyrrolidon und 3 bis 20 Gew.-% Wasser enthaltenden Stoffstrom e1, wobei das selektive Lösungsmittel, vorzugsweise N-Methylpyrrolidon, Wasser sowie Butene, Butadien, Butan und gegebenenfalls Kohlenstoffoxide enthaltende Stoffstrom d1 in einem selektiven Lösungsmittel, vorzugsweise N-Methylpyrrolidon, Wasser sowie Butan, Butene, Butadien enthaltenden Stoffstrom e2 sowie einen im Wesentlichen Butan und gegebenenfalls Kohlenstoffoxide enthaltenden Stoffstrom e3 getrennt wird;
F) Destillation des selektiven Lösungsmittels, vorzugsweise N-Methylpyrrolidon, Wasser, Butan sowie Butene, Butadien enthaltenden Stoffstroms e2 in einen im Wesentlichen selektiven Lösungsmittel, vorzugsweise N-Methylpyrrolidon und Wasser enthaltenden Stoffstrom e1 und einen Butan, Butene, Butadien enthaltenden Stoffstrom f;
G) Einspeisung des Stoffstroms f und eines sauerstoffhaltigen Gases in mindestens eine zweite Dehydrierzone und oxidative Dehydrierung von 1-Buten und 2-Butenen, wobei ein Gasstrom g enthaltend n-Butan, nicht umgesetzte(s) 1-Buten und 2-Butene, Butadien, Wasserdampf, gegebenenfalls Kohlenstoffoxide, gegebenenfalls Wasserstoff und gegebenenfalls Inertgase erhalten wird.

Kohlenstoffoxide sind Kohlendioxid, Kohlenmonoxid oder Gemische davon. Vorzugsweise wird der Stoffstrom d2 ganz oder teilweise in die erste Dehydierzone B) zurückgeführt.

Dabei kann in Stufe G) ein zusätzlicher Feedstrom eingespeist werden.

Vorzugsweise wird der Stoffstrom e1 ganz oder teilweise in die Absorptionszone D) und die Extraktionsdestillationszone E) zurückgeführt.

Vorzugsweise wird der Stoffstrom e3 ganz oder teilweise in Stufe A) zurückgeführt.

Vorzugsweise wird nach Stufe G) die folgende Stufe H) durchgeführt:
H) Entfernung des in dem Gasstrom g enthaltenen Restsauerstoffs mittels katalytischer Verbrennungsstufe, in der der Sauerstoff (bis auf geringe Spuren) mit einem Teil des oder dem gesamten zuvor abgetrennten Wasserstoff (s) d2 und/oder zusätzlich eingespeistem Wasserstoff umgesetzt wird unter Erhalt eines sauerstoffabgereicherten Stoffstromes h.

Vorzugsweise werden nach Stufe G) oder H) die folgenden Stufen I) bis L) durchgeführt:
I) Kompression in mindestens einer ersten Kompressionsstufe und Abkühlung des sauerstoffabgereicherten Stoffstromes h oder Gasstroms g, wobei mindestens ein Kondensatstrom i1 enthaltend Wasser und ein Gasstrom i2 enthaltend n-Butan, 1-Buten, 2-Butene, Butadien, Wasserstoff, Wasserdampf, gegebenenfalls Kohlenstoffoxide und gegebenenfalls Inertgase erhalten wird;
J) Abtrennung der nicht kondensierbaren und leicht siedenden Gasbestandteile, umfassend Wasserstoff, Sauerstoff, Kohlenstoffoxide, die leicht siedenden Kohlenwasserstoffe Methan, Ethan, Ethen, Propan, Propen und Inertgase als Gasstrom j2 aus dem Gasstrom i2, wobei ein C4-Produktgasstrom j1 erhalten wird, der im Wesentlichen aus C4-Kohlenwasserstoffen besteht;
K) Auftrennung des Gasstroms j1 durch Extraktivdestillation mit einem selektiven Lösungsmittel, vorzugsweise einem 80 bis 97 Gew.-% N-Methylpyrrolidon und 3 bis 20 Gew.-% Wasser enthaltendem Gemisch in einen im Wesentlichen aus Butadien und selektivem Lösungsmittel, vorzugsweise N-Methylpyrrolidon bestehenden oder diese enthaltenden Stoffstrom k1 und einen n-Butan, Butene, Wasserdampf und gegebenenfalls Inertgase enthaltenden Stoffstrom k2.
L) Destillation des selektiven Lösungsmittels, vorzugsweise N-Methylpyrrolidon, Wasser und Butadien enthaltenden Stoffstroms k1 in einem im Wesentlichen selektiven Lösungsmittell, vorzugsweise N-Methylpyrrolidon und Wasser enthaltenden Stoffstrom I1 und einen Butadien enthaltenden Stoffstrom 12.

Vorzugsweise wird nach Stufe L) die folgende Stufe M) durchgeführt:
M) Reindestillation des Butadien enthaltenden Stoffstromes I2 in einer oder zwei Kolonnen, bei der ein Butadien enthaltender Strom m2 gewonnen wird und ein gegenüber Butadien leichter flüchtige Verunreinigungen enthaltender Gasstrom m1 und/oder ein gegenüber Butadien schwerer flüchtige Verunreinigungen enthaltender Sumpfstrom m3 abgetrennt werden.

Vorzugsweise wird der Gasstrom j2 ganz oder teilweise in die zweite Dehydrierzone G) zurückgeführt.

Vorzugsweise wird der Stoffstrom k2 ganz oder teilweise in den Einsatzgasstrom in Stufe A), die Absorptionsstufe D), die Extraktionsstufe E) und/oder teilweise die zweite Dehydrierzone G) zurückgeführt.

Vorzugsweise wird die Abtrennung in Stufe J) zweistufig durch Absorption mit nachfolgender Desorption durchgeführt.

Vorzugsweise wird der Stoffstrom I1 ganz oder teilweise in die Stufe K) zurückgeführt.

Vorzugsweise wird die nicht-oxidative, katalytische Dehydrierung von n-Butan autotherm unter Einspeisung eines sauerstoffhaltigen Gases durchgeführt. Das sauerstoffhaltige Gas kann dabei beispielsweise Luft, mit Sauerstoff angereicherte Luft oder technisch reiner Sauerstoff sein.

Der n-Butan enthaltene Einsatzstrom a kann dabei aus liquefied petroleum gas (LPG) gewonnen sein.

Die Erfindung betrifft auch ein Verfahren zur Herstellung von Butenen aus n-Butan, bei dem die Verfahrensschritte A) bis F), wie sie vorstehend angegeben sind, durchgeführt werden.

Das erfindungsgemäße Verfahren erlaubt eine optimale Nutzung des eingesetzten Butans und einen optimierten Betrieb einer zweiten Dehydrierstufe durch die angegebene Aufarbeitung nach der ersten Dehydrierstufe.

Für die vorliegende Trennaufgabe sind selektive Lösungsmittel geeignet, deren Affinität zu C₄-Kohlenwasserstoffen mit Einfachbindungen in Richtung zu C₄-Kohlenwasserstoffen mit Doppelbindungen und weiter zu konjugierten Doppelbindungen und Dreifachbindungen zunimmt, bevorzugt dipolare, besonderes bevorzugt dipolar-aprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im Allgemeinen werden alkylsubstituierte niedere aliphatische Säureamide oder N-alkylsubstituierte cyclische Säureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, vorliegend abgekürzt als NMP bezeichnet, bevorzugt in wäßriger Lösung, vorteilhaft mit 0 bis 20 Gew.-% Wasser.

Erfindungsgemäß werden bevorzugt sowohl als Lösungsmittel für die Absorption in Schritt D) als auch als Extraktionsmittel für die Extraktion in Schritt E) und in Schritt K) eine Mischung aus 80 bis 97 Gew.-% N-Methylpyrrolidon und 3 bis 20 Gew.-% Wasser, bevorzugt eine Mischung aus 90 bis 93 Gew.-% N-Methylpyrrolidon und 7 bis 10 Gew.-% Wasser und insbesondere eine Mischung aus 91 bis 92 Gew.-% N-Methylpyrrolidon und 8 bis 9 Gew.% Wasser, beispielsweise eine Mischung aus 91,7 Gew.-% N-Methylpyrrolidon und 8,3 Gew.-% Wasser eingesetzt.

Das Verfahren ist im Vergleich zur Erzeugung von Butadien durch Steamcracken durch eine hohe Selektivität gekennzeichnet. Es fallen keine unerwünschten Koppelprodukte an. Es entfällt die aufwendige Abtrennung von Butadien aus dem Produktgasgemisch des Crackprozesses.

Das erfindungsgemäße Verfahren zeichnet sich durch eine besonders effektive Ausnutzung der Rohstoffe aus. So werden Verluste des Rohstoffs n-Butan durch bevorzugte Rückführung von nicht umgesetztem n-Butan aus der Verfahrensstufe E) in die erste Dehydrierungsstufe minimiert. Das n-Butan wird nicht (vollständig) durch die Verfahrensschritte F) - J) geführt, wodurch diese Apparate kleiner ausgeführt werden können.

Durch die Auftrennung von Buten und Butan nach der ersten Dehydrierstufe und bevorzugte Rückführung des Butans wird ein höherer Umsatz von Butan zu Buten erzielt, als bei Rückführung eines Butan/Butengemisches aus der Extraktvidestillation nach der zweiten Dehydrierstufe. Nicht umgesetztes Buten wird bevorzugt in die zweite Dehydrierungsstufe rückgeführt. Die teilweise Abtrennung eines butenhaltigem Produktstromes mit durch die Extraktivdestillation einstellbarem Butengehalt ist hier möglich.

Es ist außerdem möglich, in die oxidative Dehydrierungsstufe G) einen butenhaltigen C₄-Strom zusätzlich zum Strom f einzuspeisen. Dieser Strom kann aus allen butenhaltigen Quellen stammen. Denkbar sind z. B. FCC-Produktströme und durch Dimerisierung von Ethylen erzeugte butenhaltige Stoffströme.

Die Durchführung einzelner Stufen kann wie in DE-A-10 2004 059 356, DE-A-10 2004 054 766, bzw. DE-A-10 2004 061 514 beschrieben, erfolgen.

Nachstehend werden bevorzugte Verfahrensweisen beschrieben:
In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im Wesentlichen gesättigte C₂-C₅-Kohlenwasserstoffe. Daneben enthält es auch Methan und Spuren von C₅⁺-Kohlenwasserstoffen. Die Zusammensetzung von LPG kann stark schwanken. Vorteilhafter Weise enthält das eingesetzte LPG mindestens 10 Gew.-% n-Butan.

Alternativ kann ein veredelter C4-Strom aus Crackern oder Raffinerien eingesetzt werden.

In einer Variante des erfindungsgemäßen Verfahrens umfasst die Bereitstellung des n-Butan enthaltenden Dehydrier-Einsatzgasstromes die Schritte
A1) Bereitstellung eines liquefied petroleum gas (LPG)-Stroms,
A2) Abtrennung von Propan und gegebenenfalls Methan, Ethan und C5+-Kohlenwasserstoffen (hauptsächlich Pentane, daneben Hexane, Heptane, Benzol, Toluol) aus dem LPG-Strom, wobei ein Butane (n-Butan und Isobutan) enthaltender Strom erhalten wird,
A3) Abtrennung von Isobutan aus dem Butane enthaltenden Strom, wobei der n-Butan enthaltende Einsatzgasstrom erhalten wird, und gegebenenfalls Isomerisierung des abgetrennten Isobutans zu einem n-Butan/Isobutan-Gemisch und Rückführung der n-Butan/Isobutan-Gemische in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan beispielsweise in einer üblichen Rektifizierkolonne abgetrennt wird. Der verbleibende, n-Butan enthaltende Strom wird als Einsatzgasstrom für die nachfolgende Butan-Dehydrierung eingesetzt.

Der abgetrennte Isobutan-Strom kann einer Isomerisierung unterworfen werden. Dazu wird der Isobutan enthaltende Strom in einen Isomerisierungsreaktor eingespeist. Die Isomerisierung von Isobutan zu n-Butan kann wie in GB-A 2018815 beschrieben durchgeführt werden. Es wird ein n-Butan/Isobutan-Gemisch erhalten, das in die n-Butan/Isobutan-Trennkolonne eingespeist wird.

Der abgetrennte Isobutan-Strom kann auch einer weiteren Verwendung zugeführt werden, beispielsweise zur Herstellung von Methacrylsäure, Polyisobuten oder Methyl-tert.-butylether.

Der n-Butan enthaltende Einsatzgasstrom a enthält im Allgemeinen mindestens 60 Gew.-% n-Butan, vorzugsweise mindestens 90 Gew.-% n-Butan. Daneben kann er als Nebenbestandteile noch C₁-C₄-Kohlenwasserstoffe enthalten.

In einem Verfahrensteil B wird der n-Butan enthaltende Einsatzgasstrom in eine Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Butenen dehydriert, wobei auch Butadien (1,3-Butadien) gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Inertgase, wie Stickstoff im Produktgasgemisch der nicht-oxidativen katalytischen n-Butan-Dehydrierung enthalten sein. Daneben liegt im Produktgasgemisch nicht umgesetztes n-Butan vor.

Ein Merkmal der nicht-oxidativen Fahrweise gegenüber einer oxidativen Fahrweise ist, dass bei der oxidativen Dehydrierung kein freier Wasserstoff gebildet wird.

Die nicht-oxidative katalytische n-Butan-Dehydrierung kann grundsätzlich in allen aus dem Stand der Technik bekannten Reaktortypen und Fahrweisen durchgeführt werden. Eine Beschreibung von erfindungsgemäß geeigneten Dehydrierverfahren enthält auch "Catalytica® Studies Division, Oxidative Dehydrogenation and Alternative Dehydrogenation Processes" (Study Number 4192 OD, 1993,430 Ferguson Drive, Mountain View, California, 94043-5272, USA).

Die nicht-oxidative katalytische Butan-Dehydrierung kann mit oder ohne sauerstoffhaltigem Gas als Co-Feed durchgeführt werden. Vorzugsweise wird sie als autotherme nicht-oxidative Dehydrierung unter Einspeisung von Sauerstoff als Co-Feed durchgeführt. Bei der autothermen Fahrweise wird die benötigte Wärme direkt im Reaktorsystem durch Verbrennung von Wasserstoff und/oder Kohlenwasserstoffen in Gegenwart von Sauerstoff erzeugt. Vorzugsweise kann zusätzlich ein Wasserstoff enthaltender Co-Feed zugemischt werden. Dem Reaktionsgasgemisch der n-Butan-Dehydrierung wird in mindestens einer Reaktionszone zusätzlich Sauerstoff zugemischt und der in dem Reaktionsgasgemisch enthaltene Wasserstoff und/oder Kohlenwasserstoff wird zumindest teilweise verbrannt, wodurch zumindest ein Teil der benötigten Dehydrierwärme in der mindestens einen Reaktionszone direkt in dem Reaktionsgasgemisch erzeugt wird. Bevorzugt ist die Fahrweise mit reinem Sauerstoff. Sauerstoff kann bevorzugt als Sauerstoff-Dampf-Gemisch oder als Luft-Dampf-Gemisch eingespeist werden. Durch die Verwendung eines Sauerstoff-Dampfgemisches werden nur geringe Mengen an Inertgasen (Stickstoff) in den Gesamtprozess eingeschleust.

Im Allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol.

Der zur Wärmeerzeugung verbrannte Wasserstoff ist der bei der katalytischen Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte so viel Wasserstoff zugegen sein, dass das Molverhältnis im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein spezieller Oxidationskatalysator erforderlich ist. Geeignete Katalysatoren sind zum Beispiel in DE-A 10 2004 061 514 beschrieben.

Als Reaktor sind alle dem Fachmann bekannten Reaktoren zum Einsatz heterogener Katalysatoren für Gas-Feststoff-Katalyse geeignet.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Geeignete Katalysatoren sind zum Beispiel in DE-A 10 2004 061 514 beschrieben, siehe auch WO 2009/124974 und WO 2009/124945. Die Dehydriertemperatur beträgt im allgemeinen 400 bis 1100 °C, der Druck im Ausgang des Reaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Auch andere Reaktoren, wie Monolithreaktoren, sind geeignet.

Bevorzugt ist ein Reaktor (1) in Form eines liegenden Zylinders oder Prismas zur Durchführung einer autothermen Gasphasendehydrierung eines butanhaltigen Gasstromes (2) mit einem Sauerstoff enthaltenden Gasstrom (3) unter Erhalt eines Reaktionsgasgemisches, an einem heterogenen Katalysator, der als Monolith (4) ausgebildet ist, wobei
- der Innenraum des Reaktors (1) durch ein in Längsrichtung des Reaktors (1) angeordnetes kreiszylindrisches oder prismatisches gasdichtes Gehäuse G in
- einen Innenbereich A, mit einer oder mehreren katalytisch aktiven Zonen (5), worin jeweils eine Packung aus aufeinander, nebeneinander und hintereinander gestapelten Monolithen (4) und vor jeder katalytisch aktiven Zone (5) jeweils eine Mischzone (6) mit festen Einbauten vorgesehen ist und
- einen koaxial zum Innenbereich A angeordneten Außenbereich B aufgeteilt ist, und wobei
- an einem Reaktorende im Anschluss an das Gehäuse G ein Wärmetauscher (12) vorgesehen ist
- mit einer oder mehreren Zuführleitungen (7) für den zu dehydrierenden butanhaltigen Gasstrom (2),
- mit einer oder mehreren, unabhängig voneinander regelbaren Zuführleitungen (9), wobei jede Zuführleitung (9) eine oder mehrere Verteilerkammern (10) versorgt, für den Sauerstoff enthaltenden Gasstrom (3) in jede der Mischzonen (6) sowie
- mit einer Abführleitung (11) für das Reaktionsgasgemisch der autothermen Gasphasendehydrierung, wobei
- der Außenbereich B mit einem unter den Reaktionsbedingungen der autothermen Gasphasendehydrierung inerten Gas beaufschlagt ist und
- der zu dehydrierende butanhaltige Gasstrom (2) über ei-ne Zuführleitung (7) in den Wärmetauscher (12) eingeleitet, im Wärmetauscher (12) durch das Reaktionsgasgemisch im Gegenstrom durch indirekten Wärmetausch aufgeheizt und weiter an das dem Wärmetauscher (12) entgegengesetzte Ende des Reaktors geleitet, dort umgelenkt, über einen Strömungsgleichrichter (8) in den Innenbereich A eingeleitet, und in den Mischzonen (6) mit dem Sauerstoff enthaltenden Gasstrom (3) vermischt wird, worauf im Innenbereich A des Reaktors (1) die autotherme Gasphasendehydrierung stattfindet.

Bevorzugt ist dabei das unter den Reaktionsbedingungen der autothermen Gasphasendehydrierung inerte Gas Wasserdampf ist.

Bevorzugt wird dabei das unter den Reaktionsbedingungen der autothermen Gasphasendehydrierung inerte Gas als Purge-Gasstrom mit einem Massenstrom von 1/5 bis 1/100, bevorzugt 1/10 bis 1/50, bezogen auf den Massenstrom des butanhaltigen Gasstromes (2) unter geringem Überdruck von 2 bis 50 mbar, bevorzugt von 25 bis 30 mbar, bezogen auf den Druck im Innenbereich A durch den Außenbereich B geleitet, bevorzugt, indem der Purge-Gasstrom an einem Reaktorende über eine oder mehrere Zuführleitungen (20) in den Außenbereich B des Reaktors eingeleitet und am entgegengesetzten Reaktorende in den Innenbereich A des Reaktors weitergeleitet wird, insbesondere über eine oder mehrere, vorteilhaft in einem Winkel verschieden von 90° zur Zuführleitung (7) für den zu dehydrierenden butanhaltigen Gasstrom (2) angeordnete Verbindungsleitung(en) (21).

Bevorzugt wird dabei der zu dehydrierende butanhaltige Gasstrom (2), an zwei oder mehreren Stellen in den Wärmetauscher (12) eingeleitet, bevorzugt als ein Hauptstrom mit höherem Massenstrom und ein oder mehrere Nebenströme mit niedrigerem Massenstrom gegenüber dem Hauptstrom.

Bevorzugt sind dabei zusätzlich zum Wärmetauscher (12) eine oder mehrere Zusatzheizungen für den zu dehydrierenden butanhaltigen Gasstrom (2) vorgesehen.

Bevorzugt ist dabei als Zusatzheizung für den butanhaltigen Gasstrom (2) die Zuführung von Wasserstoff über eine Leitung (23) in die Zuführleitung (7) für den zu dehydrierenden butanhaltigen Gasstrom (2), möglichst nahe am Eintritt in die Mischzonen (6), die vor jeder katalytisch aktiven Zone (5) angeordnet sind, vorgesehen, wobei als Zusatzheizung eine elektrische Heizung (22) vorgesehen sein kann, die bevorzugt lösbar, als Einstecksystem, innerhalb des Außenbereichs B des Reaktors (1) eingebracht wird, oder als Muffelbrenner (22), in die Zuführleitung (7) für den zu dehydrierenden butanhaltigen Gasstrom (2) nach dem Austritt desselben aus dem Wärmetauscher (12).

Bevorzugt sind dabei im Innenbereich A zwei oder mehrere katalytisch aktive Zonen (5) mit jeweils einer Packung aus aufeinander, nebeneinander und hintereinander gestapelten Monolithen (4) vorgesehen.

Zwei oder mehrere der Reaktoren (1) können eingesetzt werden, wobei mindestens ein Reaktor (1) für die autotherme Gasphasendehydrierung genutzt und gleichzeitig mindestens ein weiterer Reaktor (1) regeneriert wird.

Die Regenerierung wird bevorzugt in einem Temperaturbereich zwischen 550 und 700°C durchgeführt.

Die Regenerierung wird bevorzugt mit einem sauerstoffhaltigen Gasstrom, enthaltend 0,1 bis 1,5 Gew.-% Sauerstoff, bezogen auf das Gesamtgewicht des sauerstoffhaltigen Gasstromes, durchgeführt.

In diesem Reaktor ist
- der Innenraum des Reaktors durch ein lösbar, in Längsrichtung des Reaktors angeordnetes kreiszylindrisches oder prismatisches, gasdichtes Gehäuse G in
- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt, wobei
   - an einem Reaktorende im Anschluss an das Gehäuse G ein Wärmetauscher vorgesehen ist
   - mit einer oder mehreren Zuführleitungen für den zu dehydrierenden butanhaltigen Gasstrom,
   - mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
   - mit einer Abführleitung für das Reaktionsgasgemisch der autothermen Gasphasendehydrierung,
wobei der Außenbereich B mit einem unter den Reaktionsbedingungen der autothermen Gasphasendehydrierung inerten Gas beaufschlagt ist und der zu dehydrierende butanstoffhaltige Gasstrom über eine Zuführleitung in den Wärmetauscher eingeleitet, durch das Reaktionsgasgemisch im Gegenstrom durch indirekten Wärmetausch aufgeheizt und weiter an das dem Wärmetauscher entgegengesetzte Ende des Reaktors geleitet, dort umgelenkt, über einen Strömungsgleichrichter in den Innenbereich A eingeleitet, und in den Mischzonen mit dem Sauerstoff enthaltenden Gasstrom vermischt wird, worauf im Innenbereich A des Reaktors die autotherme Gasphasendehydrierung stattfindet.

Die autotherme Gasphasendehydrierung findet dabei an einem heterogenen Katalysator statt, der in Form von Monolithen vorliegt.

Erfindungsgemäß werden die einzelnen Monolithe nebeneinander, übereinander und hintereinander, in der erforderlichen Anzahl, um eine katalytisch aktive Zone auszufüllen, unter Ausbildung einer Packung, gestapelt.

Vor jeder Packung ist jeweils eine Mischzone mit festen Einbauten, die nicht katalytisch aktiv sind, vorgesehen. In der Mischzone erfolgt die Vermischung des butanhaltigen Gasstromes mit dem Sauerstoff enthaltenden Strom, wobei in der in Strömungsrichtung zuerst angeströmten Mischzone die Vermischung des Sauerstoff enthaltenden Gasstromes mit dem butanhaltigen Einsatzstrom erfolgt und in den darauffolgend angeströmten Mischzonen jeweils eine Zwischenspeisung eines Sauerstoff enthaltenden Gasstromes in das noch zu dehydrierende, Butan enthaltende Reaktionsgemisch erfolgt.

Der zu dehydrierende butanhaltige Gasstrom kann bevorzugt an zwei oder mehreren Stellen in den Wärmetauscher eingeleitet werden, insbesondere als ein Hauptstrom mit höherem Massenstrom und ein oder mehrere Nebenströme mit niedrigerem Massenstrom gegenüber dem Hauptstrom.

In der Zeichnung zeigen im Einzelnen:
- Figur 1: einen Längsschnitt durch eine bevorzugte Ausführungsform eines erfindungsgemäßen Reaktors in horizontaler Ebene,
- Figur 2: einen Längsschnitt durch denselben Reaktor in vertikaler Ebene,
- Figur 3: einen Querschnitt durch den in Figur 2 dargestellten Reaktor in der Ebene B-B,
- Figur 4: einen Querschnitt durch den in Figur 2 dargestellten Reaktor in der Ebene A-A und
- Figur 5: eine vergrößerte Darstellung des in Figur 1 durch Einkreisung markierten Bereichs.

In den Figuren bezeichnen gleiche Bezugszeichen jeweils gleiche oder entsprechende Merkmale.

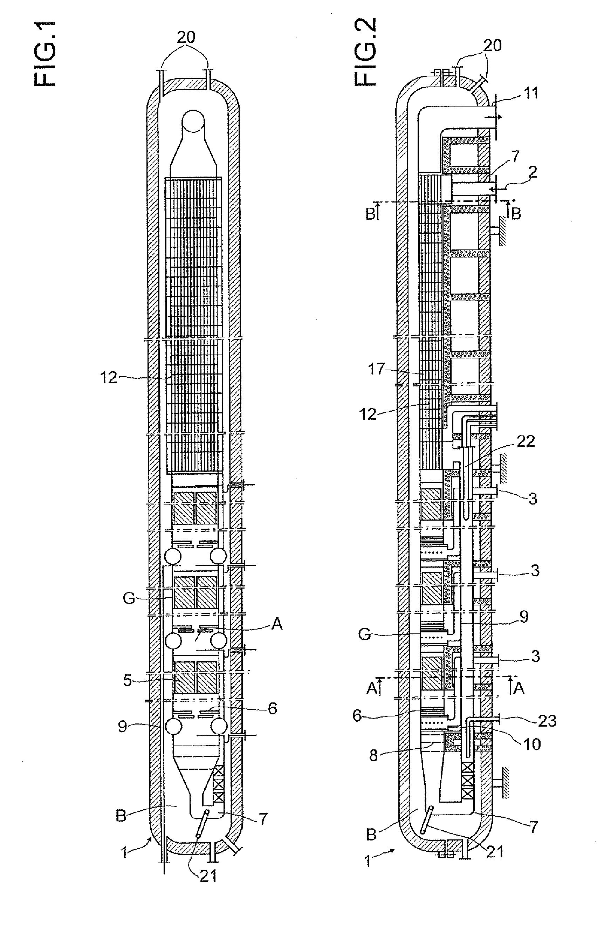

Der Längsschnitt in horizontaler Ebene in Figur 1 zeigt schematisch eine bevorzugte Ausführungsform eines Reaktors 1, der mit einem zu dehydrierenden butanhaltigen Gasstrom 2 über eine Zuführleitung 11 und über die Zuführleitungen 9 mit einem Sauerstoff enthaltenden Gasstrom 3 gespeist wird. Figur 1 zeigt, dass das Gehäuse G den Innenraum des Reaktors 1 in einen Innenbereich A und einen Außenbereich B aufteilt. Dem Innenbereich A schließt sich an einem Ende desselben ein Wärmetauscher 12 an. Auf der rechten Seite der Darstellung sind Zuführleitungen 20 für den Purge-Gasstrom und auf der linken Seite eine Verbindungsleitung 21 für den Purge-Gasstrom aus dem Außenbereich B des Reaktors in die Zuführleitung 7 für den zu dehydrierenden butanhaltigen Gasstrom 2 dargestellt. Über die Zuführleitungen 20 wird ein Purge-Gasstrom in den Außenbereich B eingeleitet, und über eine Verbindungsleitung 21 am anderen Ende des Reaktors über die Zuführleitung 7 für den zu dehydrierenden butanhaltigen Gasstrom 2 in den Innenbereich A weitergeleitet.

Die Figur 2 zeigt Zusatzheizungen, die vorteilhaft eingesetzt werden können: eine elektrische Heizung 22 sowie eine Zuführleitung 23 für Wasserstoff als Brenngas in die Zuführleitung 7 für den zu dehydrierenden butanhaltigen Gasstrom 2.

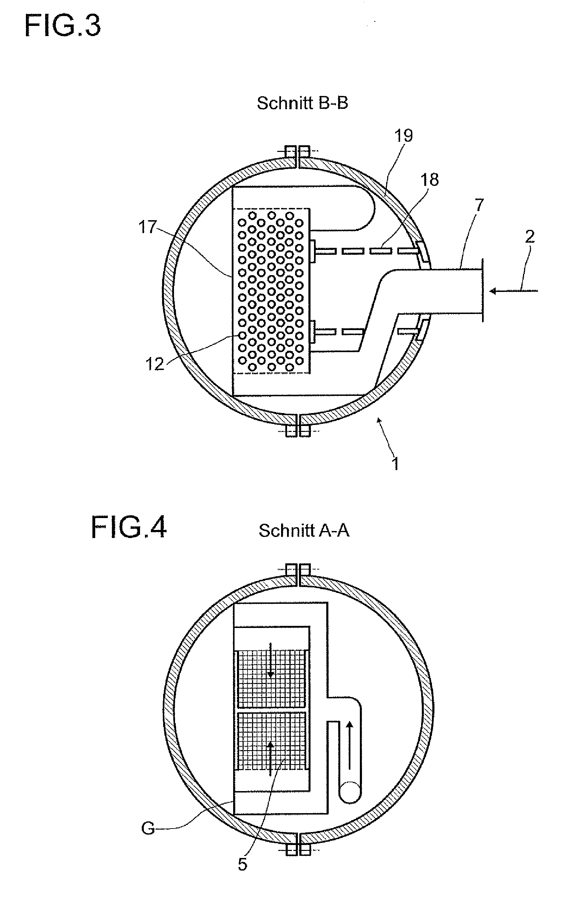

Figur 3 zeigt eine Querschnittdarstellung der Ebene B-B im Bereich des Wärmeübertragers. Die Figur verdeutlicht die Zuführung des zu dehydrierenden butanhaltigen Gasstromes 2 über die Zuführleitung 7 in den Zwischenraum zwischen den Rohren 17 der in der Figur dargestellten bevorzugten Ausführungsform eines Rohrbündelwärmetauschers 12.

In der Querschnittdarstellung ist ebenfalls die Tragekonstruktion 18 für den Rohrbündelwärmetauscher 12 zu erkennen, die bevorzugt aus Lochblechen gebildet ist, die Isolierschicht 19 der Innenwand des Reaktormantels sowie die bevorzugt als Zusatzheizung eingesetzte elektrische Heizung 22.

Figur 4 zeigt eine weitere Querschnittdarstellung in der Ebene A-A im Bereich einer Reaktionszone. Die Figur zeigt insbesondere die Zuführleitung 23 für Brenngas.

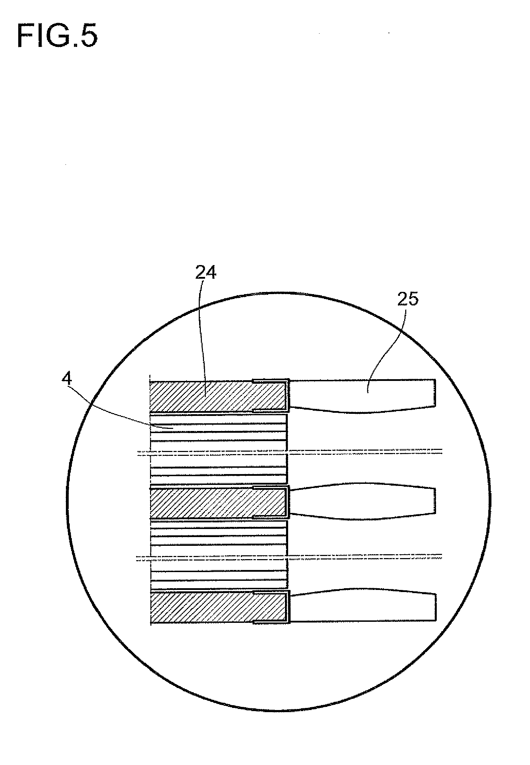

Der in Figur 5 dargestellte Ausschnitt verdeutlicht die in Verlängerung der Blähmatten 24, die in U-Profilen aus hochtemperaturfesten Geweben eingefasst sind, zugeordneten Stahlprofile 25, die mit einem Querschnitt entsprechend jenem der Blähmatten 24 an denselben ansetzen und sich zunehmend verbreitern. Die Bezugsziffer 4 in Figur 5 bezeichnet die Monolithe.

Die Butan-Dehydrierung wird allgemein bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Bei der nicht-oxidativen katalytischen n-Butan-Dehydrierung wird ein Gasgemisch erhalten, das neben Butadien 1-Buten, 2-Butene und nicht umgesetztem n-Butan in der Regel Nebenbestandteile enthält. Übliche Nebenbestandteile sind Wasserstoff, Wasserdampf, CO₂, sowie Leichtsieder (Methan, Ethan, Ethen, Propan und Propen). Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält vorzugsweise 0,1 bis 15 Vol.-% Butadien, 1 bis 15 Vol.-% 1-Buten, 1 bis 25 Vol.% 2-Buten (cis/trans-2-Buten), 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.% Wasserstoff, 0 bis 10 Vol.-% Inertgas (Stickstoff) und 0 bis 5 Vol.-% Kohlenstoffoxide, wobei die Gesamtmenge der Inhaltsstoffe 100 Vol-% beträgt.

Der die erste Dehydrierzone verlassende Produktgasstrom b kann nach Kompression in Verfahrensstufe C) in Verfahrensstufe D) in zwei Teilströme aufgetrennt werden, wobei nur einer der beiden Teilströme den weiteren Verfahrensteilen E) bis M) unterworfen wird und der zweite Teilstrom in die erste Dehydrierzone zurückgeführt wird. Eine entsprechende Verfahrensweise ist in DE-A 102 11 275 beschrieben. Es kann jedoch auch der gesamte Produktgasstrom b der nicht-oxidativen katalytischen n-Butan-Dehydrierung den weiteren Verfahrensteilen E) bis M) unterworfen werden.

In der Verfahrensstufe C) wird der Gasstrom b vorzugsweise zunächst abgekühlt. Die Abkühlung des verdichteten Gases erfolgt mit Wärmetauschern, die beispielsweise als Rohrbündel-, Spiral- oder Plattenwärmetauscher ausgeführt sein können. Die dabei abgeführte Wärme wird bevorzugt zur Wärmeintegration im Verfahren genutzt. Anschließend wird in einer bevorzugten Ausführungsform des Verfahrensschrittes C) aus dem Produktstrom Wasser abgetrennt. Die Abtrennung des Wassers erfolgt dabei vorzugsweise in einem Quench.

Anschließend wird der Gasstrom c in mindestens einer ersten Kompressionsstufe komprimiert und nachfolgend abgekühlt, wobei mindestens ein Kondensatstrom c1 enthaltend Wasser auskondensiert und ein Gasstrom c2 enthaltend n-Butan, 1-Buten, 2-Butene, Butadien, Wasserstoff, Wasserdampf, in geringen Mengen Methan, Ethan, Ethen, Propan und Propen gegebenenfalls Kohlenstoffoxide und gegebenenfalls Inertgase verbleibt.

Die Kompression kann ein- oder mehrstufig erfolgen. Insgesamt wird von einem Druck im Bereich von 1,0 bis 4,0 bar auf einen Druck im Bereich von 3,5 bis 20 bar komprimiert. Nach jeder Kompressionsstufe folgt eine Abkühlstufe, in der der Gasstrom auf eine Temperatur im Bereich von 15 bis 60°C abgekühlt wird. Der Kondensatstrom c1 kann somit bei mehrstufiger Kompression auch mehrere Ströme umfassen.

Der Gasstrom c2 besteht im Allgemeinen im Wesentlichen aus C₄-Kohlenwasserstoffen (im Wesentlichen n-Butan, 1-Buten und 2-Butene), Wasserstoff, Kohlendioxid und Wasserdampf. Daneben kann der Strom c2 noch Leichtsieder, Butadien und Inertgase (Stickstoff) als weitere Nebenkomponenten enthalten. Der Kondensatstrom c1 besteht im Allgemeinen zu mindestens 80 Gew.-%, vorzugsweise zu mindestens 90 Gew.-% aus Wasser und enthält daneben in geringem Umfang Leichtsieder, C₄-Kohlenwasserstoffe, Oxygenate und Kohlenstoffoxide.

Geeignete Verdichter sind beispielsweise Turbo-, Drehkolben- und Hubkolbenverdichter. Die Verdichter können beispielsweise mit einem Elektromotor, einem Expander oder einer Gas- oder Dampfturbine angetrieben werden. Typische Verdichtungsverhältnisse (Austrittsdruck: Eintrittsdruck) pro Verdichterstufe liegen je nach Bauart zwischen 1,5 und 3,0.

Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei in der Regel Kühlwasser oder Wärmeträgeröle zum Einsatz. Daneben wird bevorzugt Luftkühlung unter Einsatz von Gebläsen eingesetzt.

Die Absorption in Schritt (D) kann in jeder beliebigen, dem Fachmann bekannten geeigneten Absorptionskolonne durchgeführt werden. Bevorzugt wird die Absorption im Gegenstrom durchgeführt. Hierzu wird der Absorptionskolonne im unteren Bereich der Butene, Butadien, Butan, Wasserstoff, Inertgas (Stickstoff) und gegebenenfalls Kohlenstoffoxide enthaltende Stoffstrom zugeführt. Im oberen Bereich der Adsorptionskolonne wird der N-Methylpyrrolidon und Wasser enthaltende Stoffstrom aufgegeben.

Am Kopf der Absorptionskolonne wird ein wasserstoffreicher und/oder inertgas(stickstoff)reicher Stoffstrom d2 entnommen, der gegebenenfalls noch Reste an C4-Kohlenwasserstoffen und gegebenenfalls Kohlenstoffoxigenate enthält. Weiterhin kann dieser Strom Inerte (beispielsweise Stickstoff) und Leichtsieder (Ethan, Ethen, Propan, Propen, Methan) enthalten. Der N-Methylpyrrolidon und Wasser enthaltende Stoffstrom kühlt den zugeführten Butene und/oder Butadien, Butan, Wasserstoff und/oder Inertgas (Stickstoff) und gegebenenfalls Kohlenstoffoxide enthaltenden Stoffstrom ab und absorbiert gleichzeitig bevorzugt die C₄-Komponenten und teilweise Kohlenstoffoxide. Gegebenenfalls werden auch kleine Mengen an H₂, Inerten (N₂) und Leichtsiedern absorbiert. Dieser Strom wird am Sumpf der Absorptionskolonne abgezogen.

Die Verwendung einer Mischung von N-Methylpyrrolidon und Wasser als Lösungsmittel für die Absorption und als Extraktionsmittel in der Extraktivdestillation hat den Vorteil, dass die Siedetemperatur niedriger liegt als die Siedetemperatur bei Verwendung von reinem N-Methylpyrrolidon. Ein weiterer Vorteil ist, dass durch Zunahme des Wasseranteils in der als Lösungsmittel eingesetzten Mischung aus Wasser und N-Methylpyrrolidon die Selektivität gesteigert werden kann. Dies führt jedoch erwartungsgemäß zu einer Verringerung der Kapazität. Ein weiterer Vorteil ist die Selektivität des N-Methylpyrrolidons gegenüber Kohlenstoffoxiden, insbesondere Kohlendioxid. Dies ermöglicht zusätzlich zur Abtrennung der Kohlenwasserstoffe eine Abtrennung der Kohlenstoffoxide, insbesondere des Kohlendioxids vom Wasserstoff.

Die Absorption in Schritt D) wird im Allgemeinen bei einer Sumpftemperatur im Bereich von 30 bis 160 °C, einer Kopftemperatur im Bereich von 5 bis 60°C und bei einem Druck im Bereich von 2 bis 20 bar durchgeführt. Bevorzugt wird die Absorption bei einer Sumpftemperatur im Bereich von 30 bis 100°C, bei einer Kopftemperatur im Bereich von 25 bis 50 °C und einem Druck im Bereich von 8 bis 15 bar durchgeführt.

Die Absorptionskolonne ist vorzugsweise eine Füllkörperkolonne oder eine Packungskolonne. Es ist aber auch jede beliebige andere Kolonne, zum Beispiel eine Bodenkolonne denkbar. Eine für die Absorption geeignete Kolonne weist vorzugsweise 2 bis 40 theoretische Trennstufen, bevorzugt 5 bis 25 theoretische Trennstufen auf.

Die Temperatur des N-Methylpyrrolidon und Wasser enthaltenden Stoffstroms, z. B. e1 und/oder l1 der der Absorptionskolonne zugeführt wird, liegt vorzugsweise bei 10 bis 70°C, bevorzugt bei 20 bis 40°C. Die Temperatur des Butene, Butadien, Butan, Wasserstoff und/oder Inertgas (Stickstoff) und gegebenenfalls Kohlenstoffoxide enthaltenden Stoffstroms liegt vorzugsweise im Bereich zwischen 0 und 400 °C, insbesondere im Bereich zwischen 40 und 200 °C.

Das Verhältnis von eingesetztem N-Methylpyrrolidon zum Butene, Butadien, Butan, Wasserstoff und/oder Inertgas und gegebenenfalls Kohlenstoffoxide enthaltendem Stoffstrom liegt vorzugsweise im Bereich von 2 bis 30, mehr bevorzugt im Bereich von 4 bis 30 und insbesondere im Bereich von 4 bis 15, jeweils bezogen auf die Massen der eingesetzten Stoffströme.

Der bei der Absorption anfallende N-Methylpyrrolidon, Wasser, Butene, Butadien, Butan und Kohlenstoffoxide enthaltende Stoffstrom d1 enthält im Allgemeinen 20 bis 90 mol-% N-Methylpyrrolidon , 0 bis 50 mol-% Wasser, 0 bis 20 mol-% Butadien, 0 bis 20 mol-% 1-Buten, 0 bis 20 mol-% 2-Butene, 0 bis 50 mol-% Butan und 0 bis 20 mol-% Kohlenstoffoxide.

Der bei der Absorption erhaltene N-Methylpyrrolidon, Wasser, Butene, Butadien, Butan und Kohlenstoffoxide enthaltende Stoffstrom d1 wird dann in Schritt (E) einer Extraktivdestillation zugeführt.

Die Extraktivdestillation kann beispielsweise wie in Erdöl und Kohle Erdgas - Petrochemie Band 34 (8). Seiten 343 bis 346 oder Ullmanns Enzyklopädie der technischen Chemie. Band 9. 4. Auflage 1975. Seiten 1 bis 18 beschrieben durchgeführt werden.

In der Extraktivdestillation wird der Butene, Butadien, Butan, Methylpyrrolidon, Wasser und Kohlenstoffoxide enthaltende Stoffstrom d1 mit einem N-Methylpyrrolidon und Wasser enthaltenden Stoffstrom in einer Extraktivdestillationszone in Kontakt gebracht. Die Extraktivdestillationszone ist im Allgemeinen in Form einer Kolonne ausgeführt, die Böden, Füllkörper oder Packungen als Einbauten enthält. Die Extraktivdestillationszone weist im Allgemeinen 10 bis 70 theoretische Böden auf, damit eine hinreichend gute Trennwirkung erzielt wird. Vorzugsweise weist die Extraktionskolonne im Kolonnenkopf eine Rückwaschzone auf. Diese Rückwaschzone dient zur Rückgewinnung des in der Gasphase enthaltenen N-Methylpyrrolidons durch flüssigen Kohlenwasserstoffrücklauf, wozu die Kopffraktion zuvor kondensiert wird. Typische Temperaturen am Kopf der Kolonne liegen zwischen 30 und 60 °C.

Der Kopfproduktstrom e3 der Extraktivdestillationskolonne enthält Butan und Kohlenstoffoxide und wird gasförmig abgezogen. Neben Butan und Kohlenstoffoxide können weiterhin Butene, Wasserstoff und/oder Inertgas und andere Leichtsieder im Kopfproduktstrom enthalten sein. In einer bevorzugten Ausführungsform wird der Kopfproduktstrom e3 kondensiert, um Kohlenstoffoxide wie CO₂, sowie gegebenenfalls enthaltenen Wasserstoff und/oder Inertgas und Leichtsieder von Butan abzutrennen. Der flüssige Butanstrom kann zum Beispiel in die Dehydrierzone im Verfahrensschritt B) zurückgeführt werden.

Am Sumpf der Extraktivdestillationskolonne wird ein N-Methylpyrrolidon, Wasser, Butene, Butan und Butadien enthaltender Stoffstrom e2 gewonnen. Durch Abtrennung eines Teils des Butans über Kopf erfolgt hier eine Aufkonzentrierung der Butene im Stoffstrom e2. Der Grad der Aufkonzentrierung ist durch die Parametrisierung der Kolonne einstellbar.

Der N-Methylpyrrolidon, Wasser, Butan, Butene und Butadien enthaltende Strom e2, der am Sumpf der Extraktivdestillationskolonne gewonnen wird, wird einer Destillationskolonne F) zugeführt, in der über Kopf ein Stoffstrom f bestehend aus im wesentlichen Butenen, Butan und Butadien gewonnen werden. Am Sumpf der Destillationskolonne fällt ein N-Methylpyrrolidon und Wasser enthaltender Stoffstrom e1) an, wobei die Zusammensetzung des N-Methylpyrrolidon und Wasser enthaltenden Stoffstroms der Zusammensetzung entspricht, wie sie der Absorption und der Extraktion zugegeben wird. Der N-Methylpyrrolidon und Wasser enthaltende Stoffstrom wird vorzugsweise aufgeteilt und zurück in die Absorption im Verfahrensschritt D) und die Extraktivdestillation im Verfahrensschritt E) geleitet. Das Verhältnis des Gemischs aus Wasser und N-Methylpyrrolidon, das der Absorption zugeführt wird, zu dem Gemisch aus Wasser und N-Methylpyrrolidon und C₄, das der Extraktivdestillation zugeführt wird, liegt vorzugsweise im Bereich von 0,2 bis 20, insbesondere im Bereich von 0,3 bis 15.

Der über Kopf abgetrennte Stoffstrom f kann teilweise oder vollständig aus der Anlage abgezogen und als Produktstrom verwendet werden. Dabei kann der Butengehalt über die Betriebsweise der Extraktivdestillation eingestellt werden. Eine hohe Butenkonzentration verringert die Butanmenge die durch Verfahrensschritt G) und die nachfolgenden Verfahrensschritte geführt werden muss. Gleichzeitig wird hierdurch die Ausbeute der BDH-Stufe erhöht.

Die Extraktivdestillation wird vorzugsweise bei einer Sumpftemperatur im Bereich von 90 bis 250°C, insbesondere bei einer Temperatur im Bereich von 90 bis 210°C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Die Destillation im Verfahrensschritt (F) wird vorzugsweise bei einer Sumpftemperatur im Bereich von 100 bis 300 °C, insbesondere im Bereich von 150 bis 200°C und einer Kopftemperatur im Bereich von 0 bis 70° C, insbesondere im Bereich von 10 bis 50° C durchgeführt. Der Druck in der Destillationskolonne liegt dabei vorzugsweise im Bereich von 1 bis 10 bar. Die Destillationskolonne weist vorzugsweise 2 bis 30, insbesondere 5 bis 20 theoretische Trennstufen auf.

Neben dem aus Verfahrensschritt F) erhaltenen Stoffstrom f können auch weitere n-butenhaltige Stoffströme der ODH-Stufe in Verfahrensschritt G) zugeführt werden, wie sie beispielsweise in Raffinerien aus FCC-Einheiten oder durch Dimerisierung von Ethylen erhalten werden. Erfindungsgemäß ist die Zusetzung jedes n-butenhaltigen Stoffstromes denkbar.

Die Butendehydrierung kann als Einzelverfahren oder in Kombination mit einer Butandehydrierung betrieben werden. Die Butendehydrierung kann nicht-oxidativ oder oxidativ (mit einem O₂-reichem Gas als Oxidationsmittel) betrieben werden.

In der oxidativen (katalytischen) Dehydrierung im Verfahrensschritt G) werden im Wesentlichen 1-Buten und 2-Butene zu 1,3-Butadien dehydriert, wobei 1-Buten im Allgemeinen nahezu vollständig abreagiert.

Die oxidative Dehydrierung kann grundsätzlich in allen aus dem Stand der Technik bekannten Reaktortypen und nach bekannten Fahrweisen durchgeführt werden, beispielsweise im Wirbelbett, im Hordenofen, im Festbettrohr- oder Rohrbündelreaktor oder im Plattenwärmetauscherreaktor. Zur Durchführung der oxidativen Dehydrierung wird vorzugsweise ein Gasgemisch benötigt, das ein molares Sauerstoff: n-Butene-Verhältnis von mindestens 0,5 aufweist. Bevorzugt wird bei einem Sauerstoff: n-Butene-Verhältnis von 0,55 bis 50, vorzugsweise 0,55 bis 10, insbesondere 0,55 bis 3 gearbeitet. Zur Einstellung dieses Wertes wird in der Regel das aus der nicht-oxidativen katalytischen Dehydrierung stammende Produktgasgemisch direkt oder nach einer Aufarbeitung, bei der Butene aufkonzentriert und Wasserstoff abgetrennt werden, mit reinem Sauerstoff oder einem sauerstoffhaltigen Gas vermischt. In einer Ausführungsform des Verfahrens ist das sauerstoffhaltige Gas Luft. Das erhaltene sauerstoffhaltige Gasgemisch wird dann der Oxidehydrierung zugeführt. Alternativ und bevorzugt gegenüber Luft, ist es, zusätzlichen Stickstoff oder Magerluft mit einem Anteil von weniger als 23 Vol.-% als sauerstoffhaltiges Gas einzusetzen. In einer bevorzugten Ausführungsform wird das Abgas aus Verfahrensschritt J) Stoffstrom j2 mit dem Stoffstrom f und gegebenenfalls zusätzlichem Dampf vermischt und dem Verfahrensschritt G) zugeführt. Damit kann die zur Verdünnung des Stoffstroms f gegebenenfalls benötigte Stickstoffmenge reduziert oder überflüssig gemacht werden.

Die für die Oxidehydrierung besonders geeigneten Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten aus der 1. bis 15. Gruppe des Periodensystems, wie beispielsweise Kalium, Magnesium, Zirkon, Chrom, Nickel, Cobalt, Cadmium, Zinn, Blei, Germanium, Lanthan, Mangan, Wolfram, Phosphor, Cer, Aluminium oder Silizium.

Geeignete Katalysatoren und deren Herstellung sind beispielsweise beschrieben in US 4,423,281, US 4,336,409, DE-A-2600128 und DE-A-2440329 sowie WO 2009/124974 und WO 2009/124945.

Der Katalysator zur Oxidehydrierung wird im Allgemeinen als Formkörper mit einer mittleren Größe von über 2 mm eingesetzt. Aufgrund des zu beachtenden Druckverlustes während der Ausübung des Verfahrens sind kleinere Formkörper in der Regel ungeeignet. Als geeignete Formkörper seien beispielsweise genannt Tabletten, Zylinder, Hohlzylinder, Ringe, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Im Allgemeinen kann der verwendete Katalysator als so genannter Vollkatalysator eingesetzt werden. In diesem Fall besteht der gesamte Katalysatorformkörper aus der Aktivmasse, inklusive eventueller Hilfsmittel, wie etwa Graphit oder Porenbildner, sowie weiterer Komponenten. Des Weiteren ist es möglich, die Aktivmassen der Katalysatoren auf einen Träger, beispielsweise einen anorganischen, oxidischen Formkörper, inklusive eventueller Hilfsmittel, wie etwa Graphit oder Porenbildner, sowie weiterer Komponenten aufzubringen. Derartige Katalysatoren werden in der Regel als Schalenkatalysatoren bezeichnet.

Die Oxidehydrierung wird im Allgemeinen bei einer Temperatur von 220 bis 490 °C und bevorzugt von 250 bis 450 °C durchgeführt. Man wählt einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung aus Katalysator ab.

Der die oxidative Dehydrierung verlassende Produktgasstrom g enthält neben Butadien und nicht in Verfahrensschritt E) abgetrenntem n-Butan noch Wasserstoff, Kohlenstoffoxide und Wasserdampf. Als Nebenbestandteile kann er noch Sauerstoff, Inertgas, wie Stickstoff, Methan, Ethan, Ethen, Propan und Propen sowie sauerstoffhaltige Kohlenwasserstoffe, sogenannte Oxigenate, enthalten.

Im Allgemeinen weist der die oxidative Dehydrierung verlassende Produktgasstrom g 2 bis 40 Vol.-% Butadien, 5 bis 80 Vol.-% n-Butan, 0 bis 15 Vol.-% 2-Butene, 0 bis 5 Vol.-1-Buten, 5 bis 70 Vol.-% Wasserdampf, 0 bis 10 Vol.-% leichtsiedende Kohlenwasserstoffe (Methan, Ethan, Ethen, Propan und Propen), 0,1 bis 15 Vol.-% Wasserstoff, 0 bis 70 Vol.-% Inertgas, 0 bis 10 Vol.-% Kohlenstoffoxide, 0 bis 10 Vol.-% Sauerstoff und 0 bis 10 Vol.-% Oxygenate auf, wobei die Gesamtmenge der Inhaltsstoffe 100 Vol-% ergibt. Oxygenate können beispielsweise Furan, Essigsäure, Methacrolein, Maleinsäureanhydrid, Maleinsäure, Phthalsäureanhydrid, Propionsäure, Acetaldehyd, Acrolein, Formaldehyd, Ameisensäure, Benzaldehyd, Benzoesäure und Butyraldehyd sein. Daneben können in Spuren Acetylen, Propin und 1,2-Butadien enthalten sein.

Enthält der Produktgasstrom g mehr als nur geringfügige Spuren Sauerstoff, so wird im Allgemeinen eine Verfahrensstufe H) zur Entfernung von Rest-Sauerstoff aus dem Produktgasstrom g durchgeführt. Der Rest-Sauerstoff kann sich insoweit als störend auswirken, als er in nachgelagerten Verfahrensschritten eine Butadienperoxidbildung hervorrufen kann und als Initiator für Polymerisationsreaktionen wirken kann.

Unstabilisiertes 1,3-Butadien kann in Gegenwart von Sauerstoff gefährliche Butadienperoxide bilden. Die Peroxide sind viskose Flüssigkeiten. Ihre Dichte ist höher als die von Butadien. Da sie außerdem nur wenig in flüssigem 1,3-Butadien löslich sind, setzen sie sich auf den Böden von Lagerbehältern ab. Trotz ihrer relativ geringen chemischen Reaktivität sind die Peroxide sehr instabile Verbindungen, die sich bei Temperaturen zwischen 85 und 110 °C spontan zersetzen können. Eine besondere Gefahr besteht in der hohen Schlagempfindlichkeit der Peroxide, die mit der Brisanz eines Sprengstoffes explodieren.

Die Gefahr der Polymerbildung ist insbesondere bei der destillativen Abtrennung von Butadien (Schritte L und M) gegeben und kann dort zu Ablagerungen von Polymeren (Bildung von so genanntem "Popcorn") in den Kolonnen führen.

Vorzugsweise wird die Sauerstoffentfernung H) unmittelbar nach der oxidativen Dehydrierung G) durchgeführt. Im Allgemeinen wird hierzu eine katalytische Verbrennungsstufe durchgeführt, in der Sauerstoff mit in dieser Stufe zugesetztem Wasserstoff in Gegenwart eines Katalysators umgesetzt wird. Dieser Wasserstoff kann als Teil des Stoffstrom d2 aus Verfahrensstufe D) entnommen werden. Hierdurch wird eine Verringerung des Sauerstoffgehalts bis auf geringe Spuren erreicht.

Ein geeigneter Katalysator für die Oxidation von Wasserstoff enthält, geträgert auf α-Aluminiumoxid, 0,01 bis 0,1 Gew.-% Platin und 0,01 bis 0,1 Gew.-% Zinn bezogen auf das Gesamtgewicht des Katalysators. Platin und Zinn werden vorteilhaft in einem Gewichtsverhältnis von 1:4 bis 1:0,2 eingesetzt, bevorzugt in einem Verhältnis von 1:2 bis 1:0,5, insbesondere in einem Verhältnis von annähernd 1:1. Vorteilhaft enthält der Katalysator 0,05 bis 0,09 Gew.-% Platin und 0,05 bis 0,09 Gew.-% Zinn bezogen auf das Gesamtgewicht des Katalysators. Neben Platin und Zinn können gegebenenfalls Alkali- und/oder Erdalkalimetallverbindungen in Mengen kleiner 2 Gew.-%, insbesondere kleiner 0,5 Gew.-% verwendet werden. Besonders bevorzugt enthält der Aluminiumoxid-Katalysator ausschließlich Platin und Zinn als Aktivmetalle. Der Katalysatorträger aus α-Aluminiumoxid weist vorteilhaft eine BET-Oberfläche von 0,5 bis 15 m²/g auf, bevorzugt 2 bis 14 m²/g, insbesondere 7 bis 11 m²/g. Als Träger wird bevorzugt ein Formkörper eingesetzt. Bevorzugte Geometrien sind beispielsweise Tabletten, Ringtabletten, Kugeln, Zylinder, Sternstränge oder zahnradförmige Stränge mit Durchmessern von 1 bis 10 mm, bevorzugt 2 bis 6 mm. Besonders bevorzugt sind Kugeln oder Zylinder, insbesondere Zylinder.

Die katalytische Sauerstoffentfernung kann grundsätzlich in allen aus dem Stand der Technik bekannten Reaktortypen und Fahrweisen durchgeführt werden, beispielsweise im Wirbelbett, im Hordenofen, im Festbettrohr- oder Rohrbündelreaktor oder im Plattenwärmetauscherreaktor.

Eine weitere erfindungsgemäße Ausführungsform ist die Durchführung dieser katalytischen Reaktion zusammen mit der oxidativen Dehydrierung in Verfahrensschritt G in einem Reaktor mit 2 Katalysatoren und gegebenenfalls Zwischeneinspeisung des Verbrennungsgases nach der Dehydrierschüttung.

In der Verfahrensstufe l) wird der Gasstrom h vorzugsweise zunächst abgekühlt. Die Abkühlung des verdichteten Gases erfolgt mit Wärmetauschern, die beispielsweise als Rohrbündel-, Spiral- oder Plattenwärmetauscher ausgeführt sein können. Die dabei abgeführte Wärme wird bevorzugt zur Wärmeintegration im Verfahren genutzt. Anschließend wird in einer bevorzugten Ausführungsform des Verfahrensschrittes l) aus dem Produktstrom Wasser abgetrennt. Die Abtrennung des Wassers erfolgt dabei vorzugsweise in einem Quench. Der Quench dient zudem der Abtrennung von sauerstoffhaltigen Nebenprodukten.

Anschließend wird der Gasstrom h in mindestens einer ersten Kompressionsstufe komprimiert und nachfolgend abgekühlt, wobei mindestens ein Kondensatstrom i1 enthaltend Wasser auskondensiert und ein Gasstrom i2 enthaltend n-Butan, 1-Buten, 2-Butene, Butadien, gegebenenfalls Wasserstoff, Wasserdampf, in geringen Mengen Methan, Ethan, Ethen, Propan und Propen, Kohlenstoffoxide und Inertgase verbleibt.

Die Kompression kann ein- oder mehrstufig erfolgen. Insgesamt wird von einem Druck im Bereich von 1,0 bis 4,0 bar auf einen Druck im Bereich von 3,5 bis 20 bar komprimiert. Nach jeder Kompressionsstufe folgt eine Abkühlstufe, in der der Gasstrom auf eine Temperatur im Bereich von 15 bis 60°C abgekühlt wird. Der Kondensatstrom i1 kann somit bei mehrstufiger Kompression auch mehrere Ströme umfassen.

Der Kondensatstrom i1 besteht im Allgemeinen zu mindestens 80 Gew.-%, vorzugsweise zu mindestens 90 Gew.-% aus Wasser und enthält daneben in geringem Umfang Leichtsieder, C₄-Kohlenwasserstoffe, Oxygenate und Kohlenstoffoxide.

Geeignete Verdichter sind beispielsweise Turbo-, Drehkolben- und Hubkolbenverdichter. Die Verdichter können beispielsweise mit einem Elektromotor, einem Expander oder einer Gas- oder Dampfturbine angetrieben werden. Typische Verdichtungsverhältnisse (Austrittsdruck: Eintrittsdruck) pro Verdichterstufe liegen je nach Bauart zwischen 1,5 und 3,0.

Die Abkühlung des verdichteten Gases erfolgt mit Wärmetauschern, die beispielsweise als Rohrbündel-, Spiral- oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

Der Butadien, Butene, Butan, Wasserstoff, Inertgas und gegebenenfalls Kohlenstoffoxide sowie leicht siedende Kohlenwasserstoffe (Methan, Ethan, Ethen, Propan, Propen) enthaltende Stoffstrom i2, wird als Ausgangsstrom dem Schritt J) der Aufbereitung zugeführt.

In einer bevorzugten Ausführungsform des erfindungsgemäßen Verfahrens werden die nicht kondensierbaren oder leicht siedenden Gasbestandteile wie Wasserstoff, Sauerstoff, Kohlenstoffoxide, die leicht siedenden Kohlenwasserstoffe (Methan, Ethan, Ethen, Propan, Propen) und Inertgas, wie Stickstoff in einem Absorptions/Desorptions-Cyclus mittels eines hoch siedenden Absorptionsmittels abgetrennt, wobei ein C₄-Produktgasstrom j1 erhalten wird, der im Wesentlichen aus den C₄-Kohlenwasserstoffen besteht. Im Allgemeinen besteht der C₄-Produktgasstrom j1 zu mindestens 80 Vol.-%, bevorzugt zu mindestens 90 Vol.-%, besonders bevorzugt zu mindestens 95 Vol.-% aus den C₄-Kohlenwasserstoffen. Im Wesentlichen besteht der Strom j1 aus n-Butan, Butenen, wie 2-Butenen und Butadien.

Dazu wird in einer Absorptionsstufe der Produktgasstrom i2 nach vorheriger Wasserabtrennung mit einem inerten Absorptionsmittel in Kontakt gebracht und es werden die C₄-Kohlenwasserstoffe in dem inerten Absorptionsmittel absorbiert, wobei mit C₄-Kohlenwasserstoffen beladenes Absorptionsmittel und ein die obrigen Gasbestandteile enthaltendes Abgas j2 erhalten werden. In einer Desorptionsstufe werden die C₄-Kohlenwasserstoffe aus dem Absorptionsmittel wieder freigesetzt.

Die Absorptionsstufe in Schritt J) kann in jeder beliebigen, dem Fachmann bekannten geeigneten Absorptionskolonne durchgeführt werden. Die Absorption kann durch einfaches Durchleiten des Produktgasstroms i2 durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht-und Dünnschichtabsorber sowie Rotationskolonnen, Tellerwäscher, Kreuzschleierwäscher und Rotationswäscher in Betracht.

In einer erfindungsgemäßen Ausführungsform wird einer Absorptionskolonne im unteren Bereich der Butadien, Buten, Butan, Wasserstoff und/oder Stickstoff und gegebenenfalls Kohlendioxid enthaltende Stoffstrom zugeführt. Im oberen Bereich der Absorptionskolonne wird der Lösungsmittel und ggf. Wasser enthaltende Stoffstrom aufgegeben.

Erfindungsgemäße Absorptionsmittel sind Octane, Nonane, Decane, Undecane, Dodecane, Tridecane, Tetradecane, Pentadecane, Hexadecane, Heptadecane und Octadecane oder aus Raffineriestromen gewonnene Fraktionen, die als Hauptkomponenten die genannten linearen Alkane enthalten.

Geeignete Absorptionsmittel sind weiterhin vergleichsweise unpolare organische Losungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesaure und Phthalsäure mit geradkettigen C₁-C₈-Alkanolen, wie Benzoesaure-n-butylester, Benzoesauremethylester, Benzoesaureethylester, Phthalsauredimethylester, Phthalsaurediethylester, sowie sogenannte Wärmetrageröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%.

In einer bevorzugten Ausführungsform wird als Lösungsmittel für die Absorption in Schritt J) ein Alkangemisch, wie Tetradekan (technischer C14-C17 Schnitt) eingesetzt.

Am Kopf der Absorptionskolonne wird ein Abgasstrom j2 abgezogen, der im wesentlichen Inertgas, Kohlenstoffoxide, gegebenenfalls Butan, Butene, wie 2-Butene und Butadien ggf. Wasserstoff und leicht siedende Kohlenwasserstoffe (Methan, Ethan, Ethen, Propan, Propen) und Wasserdampf enthält. Dieser Stoffstrom j2 wird teilweise dem Verfahrensschritt G) zugeführt. Damit lässt sich der Eintrittsstrom des ODH Reaktors auf den gewünschten C4-Gehalt einstellen.

Der mit C4-Kohlenwasserstoffen beladene Lösungsmittelstrom wird in eine Desorptionskolonne geleitet. Erfindungsgemäß sind alle dem Fachmann bekannten Kolonneneinbauten für diesen Zweck geeignet. In einer Verfahrensvariante wird der Desorptionsschritt durch Entspannung und/oder Erhitzen des beladenen Lösungsmittels durchgeführt. Bevorzugte Verfahrensvariante ist die Zugabe von Strippdampf und/oder die Zufuhr von Frischdampf im Sumpf des Desorbers. Das C₄-abgereicherte Lösungsmittel kann als Gemisch gemeinsam mit dem kondensierten Dampf (Wasser) einer Phasentrennung zugeführt werden, so dass das Wasser vom Lösungsmittel abgeschieden wird. Alle dem Fachmann bekannten Apparate sind hierfür geeignet. Möglich ist zudem die Nutzung des vom Lösungsmittel abgetrennten Wassers zur Erzeugung des Strippdampfes. Bevorzugt werden 70 bis 100 Gew.-% Lösungsmittel und 0 bis 30 Gew.-% Wasser, besonders bevorzugt 80 bis 100 Gew.-% Lösungsmittel und 0 bis 20 Gew.-% Wasser, insbesondere 85 bis 95 Gew.-% Lösungsmittel und 5 bis 15 Gew.-% Wasser eingesetzt.

Die Abtrennung J) ist im Allgemeinen nicht ganz vollständig, so dass in dem C4-Produktgasstrom - je nach Art der Abtrennung - noch geringe Mengen oder auch nur Spuren der weiteren Gasbestandteile, insbesondere der leicht siedenden Kohlenwasserstoffe, vorliegen können. Die durch die Abtrennung J) auch bewirkte Volumenstromverringerung entlastet die nachfolgenden Verfahrensschritte.

Der im Wesentlichen aus n-Butan, Butenen, wie 2-Butenen und Butadien bestehende C4-Produktgasstrom j1 enthält im Allgemeinen 20 bis 80 Vol.-% Butadien, 20 bis 80 Vol.-% n-Butan, 0 bis 10 Vol.-% 1-Buten, und 0 bis 50 Vol.-% 2-Butene, wobei die Gesamtmenge 100 Vol.-% ergibt.

In einem Verfahrensteil K) wird der C4-Produktgasstrom j1 in einen im Wesentlichen aus n-Butan und Butenen, wie 2-Butenen bestehenden Rückführstrom k2 und einen im Wesentlichen aus Butadien bestehenden Strom k1 durch Extraktivdestillation getrennt. Der Strom k2 wird bevorzugt dem Einsatzgasstrom in Schritt A) zugegeben, in die Absorptionsstufe in Verfahrensschritt D), den Extraktionsschritt E) und/oder in Verfahrensschritt G) (ODH-Reaktor) (teilweise) zurückgeführt.

Die Extraktivdestillation K) kann beispielsweise wie in Erdöl und Kohle Erdgas - Petrochemie Band 34 (8). Seiten 343 bis 346 oder Ullmanns Enzyklopädie der technischen Chemie. Band 9. 4. Auflage 1975. Seiten 1 bis 18 beschrieben durchgeführt werden.

Die Extraktivdestillation wird vorzugsweise bei einer Sumpftemperatur im Bereich von 100 bis 250·C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

In der Extraktivdestillation wird der Butene, Butadien, Butan, Methylpyrrolidon, und Wasser enthaltende Stoffstrom mit einem wie vorstehend beschriebenen N-Methylpyrrolidon und Wasser enthaltenden Stoffstrom in einer Extraktivdestillationszone in Kontakt gebracht. Die Extraktivdestillationszone ist im Allgemeinen in Form einer oder mehrerer Kolonne(n) ausgeführt, die Böden, Füllkörper oder Packungen als Einbauten enthält. Die Extraktivdestillationszone weist im Allgemeinen 10 bis 70 theoretische Böden auf, damit eine hinreichend gute Trennwirkung erzielt wird. Vorzugsweise weist die Extraktionskolonne im Kolonnenkopf eine Rückwaschzone auf. Diese Rückwaschzone dient zur Rückgewinnung des in der Gasphase enthaltenen N-Methylpyrrolidons durch flüssigen Kohlenwasserstoffrücklauf, wozu die Kopffraktion zuvor kondensiert wird. Typische Temperaturen am Kopf der Kolonne liegen zwischen 30 und 60 °C.

Der Kopfproduktstrom k2 der Extraktivdestillationskolonne enthält im Wesentlichen Butan und Butene und in geringen Mengen Butadien und wird gasförmig oder flüssig abgezogen. In einer bevorzugten Ausführungsform wird der Kopfproduktstrom kondensiert, um Kohlenstoffoxide, wie CO₂ abzutrennen. Der flüssige Butan/Butenstrom kann in die Absorptionskolonne in Verfahrensschritt A), D), E) und/oder in Stufe G) zurückgeführt werden. Dadurch gelangt dieser Stoffstrom zusammen mit dem gequenchten, gekühlten, verdichteten und von Kondensat befreiten Produktgas der ersten Dehydrierstufe in die Extraktivdestillation zur Auftrennung on Butanen und Butenen. Diese Auftrennung von Butan und Buten muss dann nicht neben der Butadienabtrennung in der zweiten Extraktivdestillation durchgeführt werden.

Am Sumpf der Extraktivdestillationskolonne wird ein N-Methylpyrrolidon, Wasser, Butadien und in geringen Anteilen Butene, Butan enthaltender Stoffstrom k1 gewonnen, der einer Destillationskolonne L) zugeführt wird. In dieser wird über Kopf oder als Seitenabzug Butadien gewonnen werden. Am Sumpf der Destillationskolonne fällt ein N-Methylpyrrolidon und Wasser enthaltender Stoffstrom l1 an, wobei die Zusammensetzung des N-Methylpyrrolidon und Wasser enthaltenden Stoffstroms der Zusammensetzung entspricht, wie sie der Extraktion zugegeben wird. Der N-Methylpyrrolidon und Wasser enthaltende Stoffstrom wird bevorzugt in die Extraktivdestillation im Verfahrensschritt K) geleitet.

Die Extraktivdestillation wird vorzugsweise bei einer Sumpftemperatur im Bereich von 90 bis 250°C, insbesondere bei einer Temperatur im Bereich von 90 bis 210°C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Die Destillation im Verfahrensschritt L) wird vorzugsweise bei einer Sumpftemperatur im Bereich von 100 bis 300 °C, insbesondere im Bereich von 150 bis 200°C und einer Kopftemperatur im Bereich von 0 bis 70° C, insbesondere im Bereich von 10 bis 50° C durchgeführt. Der Druck in der Destillationskolonne liegt dabei vorzugsweise im Bereich von 1 bis 10 bar. Die Destillationskolonne weist vorzugsweise 2 bis 30, insbesondere 5 bis 20 theoretische Trennstufen auf.

Eine weitere Destillation in Verfahrensschritt M dient der weiteren Aufreinigung des Butadiens und kann nach dem Stand der Technik betrieben werden.

## Patentansprüche

1. Verfahren zur Herstellung von Butadien aus n-Butan mit den Schritten
A) Bereitstellung eines n-Butan enthaltenden Einsatzgasstroms a;
B) Einspeisung des n-Butan enthaltenden Einsatzgasstroms a in mindestens eine erste Dehydrierzone und nicht-oxidative, katalytische Dehydrierung von n-Butan, wobei ein Gasstrom b enthaltend n-Butan, 1-Buten, 2-Butene, Butadien, Wasserstoff, gegebenenfalls Wasserdampf, gegebenenfalls Kohlenstoffoxide und gegebenenfalls Inertgase erhalten wird;
C) Kompression in mindestens einer ersten Kompressionsstufe und Abkühlung des Gasstroms b, wobei mindestens ein Kondensatstrom c1 enthaltend Wasser und ein Stoffstrom c2 enthaltend Butene und Butadien, n-Butan, Wasserstoff, Wasserdampf, gegebenenfalls Kohlenstoffoxide und gegebenenfalls Inertgase erhalten wird;
D) Absorption der Butene und des Butadien, n-Butan, Wasserstoff, Wasserdampf, gegebenenfalls Inertgase und gegebenenfalls Kohlenstoffoxide enthaltenden Stoffstroms c2 mit einem selektiven Lösungsmittel, wie einem 80 bis 97 Gew.-% N-Methylpyrrolidon und 3 bis 20 Gew.-% Wasser enthaltenen Gemisch, wobei ein selektives Lösungsmittel, wie N-Methylpyrrolidon, Wasser sowie Butene, Butadien, Butan und gegebenenfalls Kohlendioxid enthaltender Stoffstrom d1 und ein Wasserstoff und gegebenenfalls Inertgase und Butan enthaltender Stoffstrom d2 erhalten werden;
E) Extraktivdestillation des selektiven Lösungsmittels, wie N-Methylpyrrolidon, Wasser sowie Butene, Butadien, Butan und gegebenenfalls Kohlenstoffoxide enthaltenden Stoffstrom d1 mit einem selektiven Lösungsmittel, wie einem 80 bis 97 Gew.-% N-Methylpyrrolidon und 3 bis 20 Gew.-% Wasser enthaltenden Stoffstrom e1, wobei das selektive Lösungsmittel, wie N-Methylpyrrolidon, Wasser sowie Butene, Butadien, Butan und gegebenenfalls Kohlenstoffoxide enthaltende Stoffstrom d1 in einem selektiven Lösungsmittel, wie N-Methylpyrrolidon, Wasser sowie Butan, Butene, Butadien enthaltenden Stoffstrom e2 sowie einen im Wesentlichen Butan und gegebenenfalls Kohlenstoffoxide enthaltenden Stoffstrom e3 getrennt wird;
F) Destillation des selektiven Lösungsmittels, wie N-Methylpyrrolidon, Wasser, Butan sowie Butene, Butadien enthaltenden Stoffstroms e2 in einen im Wesentlichen selektiven Lösungsmittel, wie N-Methylpyrrolidon und Wasser enthaltenden Stoffstrom e1 und einen Butan, Butene, Butadien enthaltenden Stoffstrom f;
G) Einspeisung des Stoffstroms f und eines sauerstoffhaltigen Gases in mindestens eine zweite Dehydrierzone und oxidative Dehydrierung von 1-Buten und 2-Butenen, wobei ein Gasstrom g enthaltend n-Butan, nicht umgesetzte(s) 1-Buten und 2-Butene, Butadien, Wasserdampf, gegebenenfalls Kohlenstoffoxide, gegebenenfalls Wasserstoff und gegebenenfalls Inertgase erhalten wird.

2. Verfahren nach Anspruch 1, **dadurch gekennzeichnet, dass** der Stoffstrom d2 ganz oder teilweise in die erste Dehydrierzone B) zurückgeführt wird.

3. Verfahren nach Ansprüchen 1 oder 2, **dadurch gekennzeichnet, dass** der Stoffstrom e1 ganz oder teilweise in die Absorptionsstufe D) und die Extraktivdestillationszone E) zurückgeführt wird.

4. Verfahren nach einem der Ansprüche 1 bis 3, **dadurch gekennzeichnet, dass** der Stoffstrom e3 ganz oder teilweise in Stufe A) zurückgeführt wird.

5. Verfahren nach einem der Ansprüche 1 bis 4, **dadurch gekennzeichnet, dass** nach Stufe G) die folgende Stufe H) durchgeführt wird:
H) Entfernung des in dem Gasstrom g enthaltenen Restsauerstoffs mittels katalytischer Verbrennungsstufe, in der der Sauerstoff mit einem Teil des oder dem gesamten zuvor abgetrennten Wasserstoff(s) d2 und/oder zusätzlich eingespeistem Wasserstoff umgesetzt wird unter Erhalt eines sauerstoffabgereicherten Stoffstromes h.

6. Verfahren nach einem der Ansprüche 1 bis 5, **dadurch gekennzeichnet, dass** nach Stufe G) oder H) die folgenden Stufen I) bis L) durchgeführt werden
I) Kompression in mindestens einer ersten Kompressionsstufe und Abkühlung des sauerstoffabgereicherten Stoffstromes h oder Gasstroms g, wobei mindestens ein Kondensatstrom i1 enthaltend Wasser und ein Gasstrom i2 enthaltend n-Butan, 1-Buten, 2-Butene, Butadien, Wasserstoff, Wasserdampf, gegebenenfalls Kohlenstoffoxide und gegebenenfalls Inertgase erhalten wird;
J) Abtrennung der nicht kondensierbaren und leicht siedenden Gasbestandteile, umfassend Wasserstoff, Sauerstoff, Kohlenstoffoxide, die leicht siedenden Kohlenwasserstoffe Methan, Ethan, Ethen, Propan, Propen und Inertgase als Gasstrom j2 aus dem Gasstrom i2, wobei ein C4-Produktgasstrom j1 erhalten wird, der im Wesentlichen aus C4-Kohlenwasserstoffen besteht;
K) Auftrennung des Gasstroms j1 durch Extraktivdestillation mit einem selektiven Lösungsmittel, wie 80 bis 97 Gew.-% N-Methylpyrrolidon und 3 bis 20 Gew.-% Wasser enthaltenden Gemisch in einen Butadien und selektives Lösungsmittel, wie N-Methylpyrrolidon enthaltenden Stoffstrom k1 und einen n-Butan, Butene, Wasserdampf und gegebenenfalls Inertgase enthaltenden Stoffstrom k2;
L) Destillation des selektiven Lösungsmittels, wie N-Methylpyrrolidon, Wasser und Butadien enthaltenden Stoffstroms k1 in einen im Wesentlichen selektiven Lösungsmittel, wie N-Methylpyrrolidon und Wasser enthaltenden Stoffstrom l1 und einen Butadien enthaltenden Stoffstrom 12.

7. Verfahren nach Anspruch 6, **dadurch gekennzeichnet, dass** nach Stufe L) die folgende Stufe M) durchgeführt wird
M) Reindestillation des Butadien enthaltenden Stoffstromes l2 in einer oder zwei Kolonnen, bei der ein Butadien enthaltender Strom m2 gewonnen wird und ein gegenüber Butadien leichter flüchtige Verunreinigungen enthaltender Gasstrom m1 und/oder ein gegenüber Butadien schwerer flüchtige Verunreinigungen enthaltender Sumpfstrom m3 abgetrennt werden.

8. Verfahren nach einem der Ansprüche 6 oder 7, **dadurch gekennzeichnet, dass** der Gasstrom j2 ganz oder teilweise in die zweite Dehydrierzone G) zurückgeführt wird.

9. Verfahren nach einem der Ansprüche 6 bis 8, **dadurch gekennzeichnet, dass** der Stoffstrom k2 ganz oder teilweise in den Einsatzstrom in Stufe A), die Absorptionsstufe D), die Extraktionsstufe E) und/oder teilweise die zweite Dehydrierzone G) zurückgeführt wird.

10. Verfahren nach einem der Ansprüche 6 bis 9, **dadurch gekennzeichnet, dass** die Abtrennung in Stufe J) zweistufig durch Absorption mit nachfolgender Desorption erfolgt.

11. Verfahren nach einem der Ansprüche 6 bis 10, **dadurch gekennzeichnet, dass** der Stoffstrom l1 ganz oder teilweise in die Stufe K) zurückgeführt wird.

12. Verfahren nach einem der Ansprüche 1 bis 11, **dadurch gekennzeichnet, dass** die nicht-oxidative, katalytische Dehydrierung von n-Butan autotherm unter Einspeisung eines sauerstoffhaltigen Gases durchgeführt wird.

13. Verfahren nach Anspruch 12, **dadurch gekennzeichnet, dass** als sauerstoffhaltiges Gas Luft oder mit Sauerstoff angereicherte Luft oder als sauerstoffhaltiges Gas technisch reiner Sauerstoff eingespeist wird.

14. Verfahren nach einer der Ansprüche 1 bis 13, **dadurch gekennzeichnet, dass** der n-Butan enthaltende Einsatzstrom a aus liquefied petroleum gas (LPG) gewonnen wird.

15. Verfahren nach einem der Ansprüche 1 bis 14, **dadurch gekennzeichnet, dass** ein zusätzlicher butenhaltiger Feedstrom in Stufe G) eingespeist wird.

16. Verfahren zur Herstellung von Butenen aus n-Butan, bei dem die Verfahrensschritte A) bis F) gemäß Anspruch 1 durchgeführt werden.

## Claims

1. A process for preparing butadiene from n-butane, comprising the steps of
A) providing a feed gas stream a comprising n-butane;
B) feeding the feed gas stream a comprising n-butane into at least one first dehydrogenation zone and nonoxidatively catalytically dehydrogenating n-butane to obtain a gas stream b comprising n-butane, 1-butene, 2-butenes, butadiene and hydrogen, with or without water vapor, with or without carbon oxides and with or without inert gases;
C) compressing in at least one first compression stage and cooling the gas stream b to obtain at least one condensate stream c1 comprising water and a stream c2 comprising butenes and butadiene, n-butane, hydrogen and water vapor, with or without carbon oxides and with or without inert gases;
D) absorbing the butenes and the stream c2 comprising butadiene, n-butane, hydrogen and water vapor, with or without inert gases and with or without carbon oxides, with a selective solvent such as a mixture comprising 80 to 97% by weight of N-methylpyrrolidone and 3 to 20% by weight of water, to obtain a stream d1 comprising selective solvent such as N-methylpyrrolidone, water, and butenes, butadiene and butane, with or without carbon dioxide, and a stream d2 comprising hydrogen, with or without inert gases and butane;
E) extractively distilling the stream d1 comprising selective solvent such as N-methylpyrrolidone, water, and butenes, butadiene and butane, with or without carbon oxides, with a selective solvent such as a stream e1 comprising 80 to 97% by weight of N-methylpyrrolidone and 3 to 20% by weight of water, to separate the stream d1 comprising selective solvent such as N-methylpyrrolidone, water, and butenes, butadiene and butane, with or without carbon oxides, into a stream e2 comprising selective solvent such as N-methylpyrrolidone, water, and butane, butenes and butadiene, and a stream e3 comprising essentially butane, with or without carbon oxides;
F) distilling the stream e2 comprising selective solvent such as N-methylpyrrolidone, water, butane and butenes, butadiene, to give a stream e1 comprising essentially selective solvent such as N-methylpyrrolidone and water, and a stream f comprising butane, butenes, butadiene;
G) feeding stream f and an oxygenous gas into at least one second dehydrogenation zone and oxidatively dehydrogenating 1-butene and 2-butenes to obtain a gas stream g comprising n-butane, unconverted 1-butene and 2-butenes, butadiene and water vapor, with or without carbon oxides, with or without hydrogen and with or without inert gases.

2. The process according to claim 1, wherein stream d2 is recycled fully or partly into the first dehydrogenation zone B).

3. The process according to claim 1 or 2, wherein stream e1 is recycled fully or partly into the absorption stage D) and the extractive distillation zone E).

4. The process according to any of claims 1 to 3, wherein stream e3 is recycled fully or partly into stage A).

5. The process according to any of claims 1 to 4, wherein stage G) is followed by performance of the following stage H):
H) removing the residual oxygen present in gas stream g by means of a catalytic incineration stage in which the oxygen is reacted with a portion of or all of the hydrogen d2 removed beforehand and/or additionally supplied hydrogen to obtain an oxygen-depleted stream h.

6. The process according to any of claims 1 to 5, wherein stage G) or H) is followed by performance of the following stages I) to L):
I) compressing in at least one first compression stage and cooling the oxygen-depleted stream h or gas stream g to obtain at least one condensate stream i1 comprising water and a gas stream i2 comprising n-butane, 1-butene, 2-butenes, butadiene, hydrogen and water vapor, with or without carbon oxides and with or without inert gases;
J) removing the uncondensable and low-boiling gas constituents comprising hydrogen, oxygen, carbon oxides, the low-boiling hydrocarbons methane, ethane, ethene, propane, propene and inert gases as gas stream j2 from gas stream i2 to obtain a C4 product gas stream j1 consisting essentially of C4 hydrocarbons;
K) separating gas stream j1 by extractive distillation with a selective solvent such as mixture comprising 80 to 97% by weight of N-methylpyrrolidone and 3 to 20% by weight of water into a stream k1 comprising butadiene and selective solvent such as N-methylpyrrolidone and a stream k2 comprising n-butane, butenes and water vapor, with or without inert gases;
L) distilling the selective solvent such as stream k1 comprising N-methylpyrrolidone, water and butadiene to give a stream 11 comprising essentially selective solvent such as N-methylpyrrolidone and water, and a stream 12 comprising butadiene.

7. The process according to claim 6, wherein stage L) is followed by performance of the following stage M) :
M) purifying distillation of the stream 12 comprising butadiene in one or two columns, in which a stream m2 comprising butadiene is obtained and a gas stream m1 comprising more volatile impurities than butadiene and/or a bottom stream m3 comprising less volatile impurities than butadiene is/are removed.

8. The process according to either of claims 6 and 7, wherein gas stream j2 is recycled fully or partly into the second dehydrogenation zone G).

9. The process according to any of claims 6 to 8, wherein stream k2 is recycled fully or partly into the feed stream in stage A), absorption stage D), extraction stage E) and/or partly the second dehydrogenation zone G).

10. The process according to any of claims 6 to 9, wherein the removal in stage J) is effected in two stages, by absorption with subsequent desorption.

11. The process according to any of claims 6 to 10, wherein stream 11 is recycled fully or partly into stage K).

12. The process according to any of claims 1 to 11, wherein the nonoxidative catalytic dehydrogenation of n-butane is performed autothermally with feeding of an oxygenous gas.

13. The process according to claim 12, wherein the oxygenous gas fed in is air or oxygen-enriched air, or the oxygenous gas fed in is oxygen of technical grade purity.

14. The process according to any of claims 1 to 13, wherein the feed stream a comprising n-butane is obtained from liquefied petroleum gas (LPG).

15. The process according to any of claims 1 to 14, wherein an additional butenic feed stream is fed into stage G).

16. A process for preparing butenes from n-butane, in which process steps A) to F) are conducted according to claim 1.

## Revendications

1. Procédé pour la préparation de butadiène à partir de n-butane présentant les étapes
A) mise à disposition d'un flux gazeux d'utilisation a contenant du n-butane ;
B) injection du flux gazeux d'utilisation a, contenant du n-butane, dans au moins une première zone de déshydrogénation et déshydrogénation catalytique non oxydante du n-butane, avec obtention d'un flux gazeux b, contenant du n-butane, du 1-butène, des 2-butènes, du butadiène, de l'hydrogène, le cas échéant de la vapeur d'eau, le cas échéant des oxydes de carbone et le cas échéant des gaz inertes ;
C) compression dans au moins une première étape de compression et refroidissement du flux gazeux b, avec obtention d'au moins un flux de condensat c1, contenant de l'eau, et d'un flux de substances c2, contenant des butènes et du butadiène, du n-butane, de l'hydrogène, de la vapeur d'eau, le cas échéant des oxydes de carbone et le cas échéant des gaz inertes ;
D) absorption des butènes et du flux gazeux c2, contenant du butadiène, du n-butane, de l'hydrogène, de la vapeur d'eau, le cas échéant des gaz inertes et le cas échéant des oxydes de carbone, avec un solvant sélectif, tel qu'un mélange comprenant 80 à 97% en poids de N-méthylpyrrolidone et 3 à 20% en poids d'eau, avec obtention d'un flux de substances d1, contenant du solvant sélectif, tel que la N-méthylpyrrolidone, de l'eau ainsi que des butènes, du butadiène, du butane et le cas échéant du dioxyde de carbone, et d'un flux de substances d2, contenant de l'hydrogène et le cas échéant des gaz inertes et du butane ;
E) distillation extractive du flux de substances d1, contenant du solvant sélectif, tel que la N-méthylpyrrolidone, de l'eau ainsi que des butènes, du butadiène, du butane et le cas échéant des oxydes de carbone, avec un solvant sélectif, tel qu'un flux de substances e1, contenant 80 à 97% en poids de N-méthylpyrrolidone et 3 à 20% d'eau, le flux de substances d1, contenant du solvant sélectif, tel que la N-méthylpyrrolidone, de l'eau ainsi que des butènes, du butadiène, du butane et le cas échéant des oxydes de carbone, étant séparé en un flux de substances e2, contenant du solvant sélectif, tel que la N-méthylpyrrolidone, de l'eau ainsi que du butane, des butènes, du butadiène, ainsi qu'en un flux de substances e3, contenant essentiellement du butane et le cas échéant des oxydes de carbone ;
F) distillation du flux de substances e2, contenant du solvant sélectif, tel que la N-méthylpyrrolidone, de l'eau, du butane ainsi que des butènes, du butadiène, en un flux de substances e1, contenant essentiellement du solvant sélectif, tel que la N-méthylpyrrolidone, et de l'eau, et en un flux de substances f, contenant du butane, des butènes, du butadiène ;
G) injection du flux de substances f et d'un gaz contenant de l'oxygène dans au moins une deuxième zone de déshydrogénation et déshydrogénation oxydante du 1-butène et des 2-butènes, avec obtention d'un flux gazeux g, contenant du n-butane, du 1-butène et des 2-butènes non transformés, du butadiène, de la vapeur d'eau, le cas échéant des oxydes de carbone, le cas échéant de l'hydrogène et le cas échéant des gaz inertes.

2. Procédé selon la revendication 1, **caractérisé en ce que** le flux de substances d2 est recyclé totalement ou partiellement dans la première zone de déshydrogénation B).

3. Procédé selon les revendications 1 ou 2, **caractérisé en ce que** le flux de substances e1 est recyclé totalement ou partiellement dans l'étape d'absorption D) et dans la zone de distillation extractive E).

4. Procédé selon l'une quelconque des revendications 1 à 3, **caractérisé en ce que** le flux de substances e3 est recyclé totalement ou partiellement dans l'étape A).

5. Procédé selon l'une quelconque des revendications 1 à 4, **caractérisé en ce qu'**après l'étape G), l'étape H) ci-dessous est réalisée :
H) élimination de l'oxygène résiduel contenu dans le flux gazeux g au moyen d'une étape de combustion catalytique, dans laquelle l'oxygène est transformé avec une partie ou la totalité de l'hydrogène d2 séparé au préalable et/ou de l'hydrogène injecté en plus avec obtention d'un flux de substances h appauvri en oxygène.

6. Procédé selon l'une quelconque des revendications 1 à 5, **caractérisé en ce qu'**après l'étape G) ou H), les étapes I) à L) ci-dessous sont réalisées :
I) compression dans au moins une première étape de compression et refroidissement du flux de substances h appauvri en oxygène ou du flux gazeux g, avec obtention d'au moins un flux de condensat i1, contenant de l'eau, et d'un flux gazeux i2, contenant du n-butane, du 1-butène, des 2-butènes, du butadiène, de l'hydrogène, de la vapeur d'eau, le cas échéant des oxydes de carbone et le cas échéant des gaz inertes ;
J) séparation des constituants gazeux non condensables et à bas point d'ébullition, comprenant de l'hydrogène, de l'oxygène, des oxydes de carbone, les hydrocarbures à bas point d'ébullition méthane, éthane, éthylène, propane, propène, et des gaz inertes en tant que flux gazeux j2 du flux gazeux i2, avec obtention d'un flux gazeux j1 de produits en C4, qui est essentiellement constitué d'hydrocarbures en C4 ;
K) séparation du flux gazeux j1 par distillation extractive à l'aide d'un solvant sélectif, tel qu'un mélange contenant 80 à 97% en poids de N-méthylpyrrolidone et 3 à 20% en poids d'eau, en un flux de substances k1, contenant du butadiène et du solvant sélectif, tels que la N-méthylpyrrolidone, et en un flux de substances k2, contenant du n-butane, des butènes, de la vapeur d'eau et le cas échéant des gaz inertes ;
L) distillation du flux de substances k1, contenant du solvant sélectif, tel que la N-méthylpyrrolidone, de l'eau et du butadiène, en un flux de substances 11, contenant essentiellement du solvant sélectif, tel que la N-méthylpyrrolidone, et de l'eau, et en un flux de substances 12 contenant du butadiène.

7. Procédé selon la revendication 6, **caractérisé en ce qu'**après l'étape L), l'étape M) ci-dessous est réalisée :
M) distillation pure du flux de substances 12 contenant du butadiène dans une ou deux colonnes, lors de laquelle on obtient un flux m2 contenant du butadiène et un flux gazeux m1 plus léger que le butadiène contenant des impuretés volatiles et/ou un flux de fond m3 plus lourd que le butadiène contenant des impuretés volatiles.

8. Procédé selon l'une quelconque des revendications 6 ou 7, **caractérisé en ce que** le flux gazeux j2 est recyclé totalement ou partiellement dans la deuxième zone de déshydrogénation G).

9. Procédé selon l'une quelconque des revendications 6 à 8, **caractérisé en ce que** le flux de substances k2 est recyclé totalement ou partiellement dans le flux d'utilisation dans l'étape A), dans l'étape d'absorption D), dans l'étape d'extraction E) et/ou partiellement dans la deuxième zone de déshydrogénation G).

10. Procédé selon l'une quelconque des revendications 6 à 9, **caractérisé en ce que** la séparation dans l'étape J) a lieu en deux étapes par absorption avec désorption consécutive.

11. Procédé selon l'une quelconque des revendications 6 à 10, **caractérisé en ce que** le flux de substances 11 est recyclé totalement ou partiellement dans l'étape K).

12. Procédé selon l'une quelconque des revendications 1 à 11, **caractérisé en ce que** la déshydrogénation catalytique non oxydante du n-butane est réalisée en mode autothermique avec injection d'un gaz contenant de l'oxygène.

13. Procédé selon la revendication 12, **caractérisé en ce qu'**on injecte, en tant que gaz contenant de l'oxygène, de l'air ou de l'air enrichi en oxygène ou, en tant que gaz contenant de l'oxygène, de l'oxygène techniquement pur.

14. Procédé selon l'une quelconque des revendications 1 à 13, **caractérisé en ce que** le flux d'utilisation a contenant du n-butane est obtenu à partir de gaz de pétrole liquéfié (GPL).

15. Procédé selon l'une quelconque des revendications 1 à 14, **caractérisé en ce qu'**on injecte un flux d'alimentation supplémentaire, contenant du butène, dans l'étape G).

16. Procédé pour la préparation de butènes à partir de n-butane, dans lequel les étapes de procédé A) à F) selon la revendication 1 sont réalisées.
